(19) Europäisches Patentamt
European Patent Office
Office européen des brevets

(11) EP 1 767 655 A1

(12) EUROPEAN PATENT APPLICATION
published in accordance with Art. 158(3) EPC

(43) Date of publication:
28.03.2007 Bulletin 2007/13

(21) Application number: 05762024.7

(22) Date of filing: 12.07.2005

(51) Int Cl.:
*C12Q 1/68* (2006.01) *A61K 31/517* (2006.01)
*A61K 35/48* (2006.01) *A61K 45/00* (2006.01)
*A61K 48/00* (2006.01) *A61P 37/00* (2006.01)
*A61P 43/00* (2006.01) *C07K 14/705* (2006.01)
*C12N 15/09* (2006.01) *C12N 5/06* (2006.01)
*C12Q 1/02* (2006.01) *G01N 33/15* (2006.01)
*G01N 33/50* (2006.01)

(86) International application number:
PCT/JP2005/013220

(87) International publication number:
WO 2006/006722 (19.01.2006 Gazette 2006/03)

(84) Designated Contracting States:
AT BE BG CH CY CZ DE DK EE ES FI FR GB GR HU IE IS IT LI LT LU LV MC NL PL PT RO SE SI SK TR

(30) Priority: 13.07.2004 JP 2004205877
02.11.2004 JP 2004319580

(71) Applicant: Takeda Pharmaceutical Company Limited
Osaka-shi,
Osaka 541-0045 (JP)

(72) Inventors:
- HINUMA, Shuji,
c/o TAKEDA PHARMACEUTICAL CO. LTD
Osaka-shi, Osaka 532-8686 (JP)
- HOSOYA, Masaki
Tsukuba-shi,
Ibaraki 3004293 (JP)
- SATO, Rie
Tsukuba-shi,
Ibaraki 3004293 (JP)

(74) Representative: Kalhammer, Georg et al
Lederer & Keller
Patentanwälte
Prinzregentenstrasse 16
80538 München (DE)

# (54) METHOD OF CONTROLLING CELL FUNCTIONS

(57) The present invention provides methods of screening genes associated with cell regeneration, growth and differentiation and regulators for cell regeneration, growth or differentiation; regulators for cell regeneration, growth or differentiation; and so on. More specifically, the present invention provides methods of screening genes associated with cell regeneration, growth and differentiation, which comprises the step of quantitatively analyzing expression levels of multiple genes collectively in a cell having a regeneration, differentiation or growth capability to thereby identify the genes associated with the regeneration, growth or differentiation of the cell; methods of screening genes associated with cell regeneration, growth and differentiation, which comprises the step of quantitatively analyzing expression levels of multiple genes collectively in a cell having a regeneration, differentiation or growth capability to thereby identify one or more genes that show higher expression levels in the cell or are specific in expression as compared to the other cells; and, the step of contacting at least one candidate substance capable of regulating the functions of the identified genes or their gene products with the cell and comparing changes before and after the contact; and so on.

EP 1 767 655 A1

## Description

### TECHNICAL FIELD

The present invention relates to a method of screening genes associated with cell regeneration, growth or differentiation, a method of screening regulators for cell regeneration, growth or differentiation, regulators for cell regeneration, growth or differentiation comprising substances obtained by the screening method, and so on. The present invention also relates to regulators for cell regeneration, growth or differentiation comprising cholecystokinin receptors or compounds acting on the receptor proteins, etc. The present invention further relates to a method of differentiation-inducing human mesenchymal stem cells into osteoblast cells in serum-free medium.

### BACKGROUND ART

Regeneration is a phenomenon that the lost cells/tissues in living organisms are restored by growth or differentiation of stem cells, etc. Constant cell renewal to supply new cells in place of the dead cells as in the skin or gastrointestinal epithelium is termed physiological regeneration, and regeneration to restore and replenish cells/tissues rapidly lost by injuries or disorders is termed pathological regeneration. Regeneration is an essential phenomenon that multicellular organisms survive. However, the inherent regenerative capability is limited in higher animals including human, and the organs or tissues severely damaged or damaged in a wide area beyond their regenerative capability are not repaired, which would cause danger of destroying the life support of an individual as a whole. In some organs such as kidney, liver and heart vital for the maintenance of organisms, therapy by organ transplantation has already been established on their dysfunction but due to the problems of securing donors, immunocompatibility, etc., there was a limit to the number of patients who could potentially benefit from the therapy. Recently regenerative medicine has been a focus of attention as overcoming such problems in organ transplantation. This is to develop technologies that regulate the capability inhered in living organisms in relation to the generation or regeneration of tissues and effect the tissue remodeling and organ regeneration from materials such as autologous cells or those collected from the others. Such technologies already clinically available include bone marrow transplantation performed for many disorders including leukemia, skin transplantation for burns, etc. In addition, these technologies are expected to be applied to neural stem cell transplantation for Parkinson's disease, bone marrow cell transplantation for myocardial infarction, pancreatic islet transplantation for diabetes mellitus, etc.

### DISCLOSURE OF INVENTION

In the present circumstances, there is a limit in the kind of cells or organs that can be artificially produced using stem cells (embryonic stem cells, somatic stem cells, etc.), preadipocytes, etc. Referring to many organs such as pancreatic β cells, kidney, alimentary tract, etc., production of clinically available cells or tissues has not yet been achieved.

Furthermore, even though certain cells are useful for regenerative medicine to some extent, they do not often reach the level sufficient to completely restore the lost function. In order to make cells or tissues available for regenerative medicine, which are highly demanded for clinical purposes but which preparation is impossible or less efficient, it is required to develop technologies for regulating cell differentiation more efficiently than the conventional one. Also, by applying a new mechanism for regulating cell differentiation that is unraveled in the course of developing technologies, a novel system for screening therapeutic candidates to promote tissue remodeling or organ regeneration in vivo or in vitro can be constructed. In addition, when all cells and tissues from human or higher animals can be mass-produced efficiently, these technologies become available for the screening, drug efficacy evaluation, safety studies of therapeutic candidates in conventional drug discovery processes.

In the generation or regeneration of organs, multiple phenomena including the growth and differentiation of stem cells, direct or indirect interaction with environments supporting the same or with many cells present at the vicinity, construction of functional tissue structures, etc. are mutually related to one another. In particular, both internal and external stimuli on individual cells are transduced intracellularly through binding of the molecules present on humoral factors or cells to their receptor molecules. When a receptor expressed in each cell can be identified and a natural ligand, a recombinant or synthetic agonist or antagonist, which specifically reacts with the receptor, can be acted thereon, the desired mature cells can be differentiated efficiently from stem cells or precursor cells, or conversely, dedifferentiation, transdifferentiation, etc. can also be induced. Using such a cell differentiation-regulating effect as an indicator of screening, a novel substance acting on the intracellular signal transduction system or metabolism system thereby to regulate differentiation, can also be obtained, in addition to those substances directly acting on the receptor. Besides, it is widely known that intracellular signal transduction systems mediated by various receptors interact with each other; thus by stimulating cells concurrently or consecutively via multiple receptors, different effects can be expected from those caused by a single receptor-mediated stimulation. It is known that subtypes are present in some receptors, and even though the same ligands are acted, one may act promotionally but the other suppressively. In such a case that it is desired to selectively stimulate only one activity in regulating the cell differentiation, an agonist or antagonist that selectively acts on the target receptor subtype can be reacted. The type of receptor molecules expressed on the cell surface can be used also as surface marker antigens for labeling and selecting cells in such a state that the cells are kept alive. Depending upon antibodies, some of them may recognize the binding sites for ligands or their vicinity as antigens and such antibodies can be used also as antibodies for neutralizing the action of endogenous ligands. In receptor molecules that are present in cells like nuclear receptors, recognize the particular sequence on chromosomal DNA and bind to regulate gene transcription, many are known as a group of genes to be regulated. It is thus possible to predict the effects exerted after ligand addition by detecting expression of the receptors.

As described above, receptor molecules play an important role as a switch for the generation or regeneration of organs/parts, which can be used to artificially regulate cell differentiation or growth. However, numerous receptors are actually expressed on the cell surfaces or in cells and thus a conventional method which allows a differentiation or growth factor to act on cells and evaluate its effects required enormous investigations to reach an effective receptor-ligand combination. In order to gain information as to whether a particular receptor is expressed or not, there are methods including the binding test, detection of signal transduction or activity, northern blot, RT-PCR, etc. In any of these methods, as the number of receptors under investigation increases, handling becomes complicated. It was thus difficult to even mutually compare the expression levels. It is also well known that receptors expressed in cells vary with differentiation both qualitatively and quantitatively and the sensitivity of cells to a differentiation-inducing factor also varies with differentiation. Accordingly, for the purpose of efficiently regulating the cell differentiation, it was necessary to assess the expression of receptors at each stage of differentiation broadly and quantitatively.

As a result of extensive studies, the present inventors have found that by broad and quantitative analysis of gene expression in stem cells or precursor cells, receptors expressed in the cells during the process of their differentiation, these cells can be differentiation-induced efficiently. Based on these findings, the inventors have continued further investigations and come to accomplish the present invention.

In other words, the present invention provides methods of screening genes associated with cell regeneration, growth or differentiation, methods of screening regulators for cell regeneration, growth or differentiation, regulators for cell regeneration, growth or differentiation comprising substances obtained by these screening methods, regulators for cell regeneration, growth or differentiation comprising cholecystokinin receptors or compounds acting on the receptor proteins, methods for diagnosis of disorders associated with the genes using the genes identified by the screening methods described above or their gene products, diagnostic agents or kits used for these methods, and so on, which are described below.

(1) A method of screening a gene associated with the regeneration, growth or differentiation of a cell, which comprises the step of quantitatively analyzing expression levels of multiple genes collectively in a cell having a regeneration, differentiation or growth capability to thereby identify the gene associated with the regeneration, growth or differentiation of the cell.

(2) The method according to (1) above, which comprises the step of quantitatively analyzing expression levels of multiple genes collectively in a cell having a regeneration, differentiation or growth capability to thereby identify one or more genes that show higher expression levels in the cell or are specific in expression as compared to the other cells; and, the step of contacting at least one candidate substance capable of regulating the function of the identified genes or their gene products with the cell and comparing changes before and after the contact.

(3) The screening method according to (1) above, wherein the cell having a regeneration, differentiation or growth capability is selected from an embryonic stem cell, a somatic stem cell, a primary culture cell and a cell line from human or warm-blooded animal.

(4) The method according to (1) above, wherein the multiple genes are a group of genes selected from a G protein-coupled receptor gene family; a nuclear receptor gene family; a tyrosine kinase-type receptor gene family; a transcription factor; a gene family related to any one of a protein kinase, a protein phosphatase, a protease, an ATPase, a GTPase, a DNA-bound protein, a cell adhesion factor and its receptor, an ion channel, a transporter, an extracellular matrix component, an intracellular cytoskeleton component, a cell growth factor, a cytokine, a neurotrophic factor, a physiologically active peptide, a hormone, an oxidase or reductase, a hydrolase, a hydroxylase, a methylase or demethylase, a transferase, a ribosome-constituting protein and a histocompatible antigen protein group; and a group of genes which act directly or indirectly on the above genes to regulate their activities.

(5) The method according to (1) above, wherein the multiple genes are the group of genes selected from the G protein-coupled receptor gene family, the nuclear receptor gene family and the protein kinase-type receptor gene family.

(6) The method according to (1) above, wherein an mRNA sample suspected of containing multiple target mRNAs obtained from a cell having a regeneration, differentiation or growth capability is brought in contact with each amplification reagent comprising one pair of primers corresponding to each target mRNA, respectively, at each reaction site of a reactor having multiple reaction sites for amplification; and the amounts of amplification products formed are measured to thereby identify the genes associated with the regeneration, growth or differentiation of the cell.

(7) The method according to (6) above, wherein the reactor is a plate having multiple wells as the reaction sites.

(8) The method according to (7) above, wherein the plate is a 96-well or 384-well plate.

(9) The method according to (6) above, wherein 10 to 800 pairs of primers are used.

(10) The method according to (6) above, wherein 10 to 300 pairs of primers are used.

(11) The method according to (6) above, wherein the amplification is performed by a polymerase chain reaction.

(12) The method according to (6) above, wherein the measurement of the amount of the amplification product formed is performed using a probe complementary or substantially complementary to the amplification product.

(13) The method according to (12) above, wherein the probe is a probe hybridizable to mRNA and cDNA.

(14) The method according to (12) above, wherein the probe is a fluorescence-labeled probe.

(15) The method according to (13) above, wherein an mRNA showing increased or decreased expression in the mRNA sample is identified and a gene encoding the mRNA is identified as the gene associated with the regeneration, growth or differentiation of the cell.

(16) A method of screening a regulator for the regeneration, growth or differentiation of a cell, which comprises the step of quantitatively analyzing expression levels of multiple genes collectively in a cell having a regeneration, differentiation or growth capability to thereby identify one or more genes that show higher expression levels in the cell or are specific in expression as compared to the other cells; and, the step of contacting at least one candidate substance which can regulate the function of the identified genes or their gene products with the cell and comparing changes before and after the contact.

(17) A medicament comprising an agonist, antagonist, inhibitor, activator or antibody, to the gene product of the gene identified by the screening method according to (1) above, or a polynucleotide encoding the gene product or an antisense polynucleotide thereof.

(18) The medicament according to (17) above, which is a regulator for the regeneration, growth or differentiation of a cell.

(18a) A method of regulating the regeneration, growth or differentiation of a cell comprising the gene product of the gene identified by the screening method according to (1) above, which comprises inhibiting or activating said gene product

(19) A method of regulating the regeneration, growth or differentiation of a cell, wherein the regulation is performed using the medicament according to (17) above.

(20) The method according to (19) above, wherein the cell is an embryonic stem cell, a somatic stem cell, a mesenchymal stem cell, a spleen cell, a neuronal cell, a glial cell, a pancreatic ($\alpha$, $\beta$ or $\delta$) cell, a cartilage cell, a bone cell, a bone marrow cells, a mesangial cell, an epithelial cell, an epidermal cell, an endothelial cell, a fibroblast cell, a fiber cell, a muscle (skeletal muscle/cardiac muscle) cell, a fat cell, an immune cell, an vascular endothelial cell, an endothelial progenitor cell or a hematopoietic cell.

(20a) The method according to (18a) above, wherein the cell is an embryonic stem cell, a somatic stem cell, a mesenchymal stem cell, a spleen cell, a neuronal cell, a glial cell, a pancreatic cell, a cartilage cell, a bone cell, a bone marrow cells, a mesangial cell, an epithelial cell, an epidermal cell, an endothelial cell, a fibroblast cell, a fiber cell, a muscle cell, a fat cell, an immune cell, an vascular endothelial cell, an endothelial progenitor cell or a hematopoietic cell.

(21) A method of regenerating an organ, which comprises using the medicament according to (17) above.

(21 a) A method of regenerating an organ, which comprises using the method according to (18a) above.

(22) The method according to (21) or (21a) above, wherein the organ is brain, each part of the brain, cornea, spinal marrow, pituitary, stomach, pancreas, kidney, liver, genital gland, thyroid gland, gallbladder, bone marrow, adrenal, skin, muscle, lung, digestive tract, blood vessel, heart, thymus gland, spleen, submandibular gland, peripheral blood, peripheral blood cell, prostate gland, testicle, testis, ovary, placenta, uterus, tooth, bone, joint or skeletal muscle.

(23) The medicament according to (17) above, which is a medicament for treating a disease associated with the regeneration, growth or differentiation of the cell.

(23a) The method according to (18a) above, which is a method of treating a disease associated with the regeneration, growth or differentiation of the cell according to (18a) above.

(24) The medicament according to (17) above, wherein the disease is a neurological disorder, an inflammatory disorder, a circulatory disorder, a cancer, obesity, diabetes mellitus, an immune disorder, a liver/gallbladder disorder, an alimentary disorder, heat burn, fracture, osteoarthritis, periodontal disease or alopecia.

(24a) The method according to (23 a) above, wherein the disease is a neurological disorder, an inflammatory disorder, a circulatory disorder, a cancer, obesity, diabetes mellitus, an immune disorder, a liver/gallbladder disorder, an alimentary disorder, heat burn, fracture, osteoarthritis, periodontal disease or alopecia.

(25) A method of treating a disease associated with the regeneration, growth or differentiation of a cell, which comprises administering the medicament according to (17) above.

(26) The treating method according to (25) above, wherein the disease is a neurological disorder, an inflammatory disorder, a circulatory disorder, a cancer, obesity, diabetes mellitus, an immune disorder, a liver/gallbladder disorder, an alimentary disorder, heat burn, fracture, osteoarthritis, periodontal disease or alopecia.

(27) A medicament comprising a protein comprising the same or substantially the same amino acid sequence as the amino acid sequence represented by SEQ ID NO: 1, a DNA encoding the protein, or a compound which acts on the protein.

(28) The medicament according to (27) above, comprising the compound which acts on a protein comprising the amino acid sequence represented by SEQ ID NO: 1.

(28a) A method of regulating the regeneration, growth or differentiation of a cell comprising a protein comprising the same or substantially the same amino acid sequence as the amino acid sequence represented by SEQ ID NO: 1, which comprises inhibiting or activating said protein.

(29) The medicament according to (28) above, wherein the compound is an agonist, antagonist, inhibitor, activator or antibody, to the protein.

(30) The medicament according to (27) or (29) above, which is a regulator for the regeneration, growth or differentiation of a cell.

(31) The medicament according to (28) above, wherein the compound is cholecystokinin or its analogue.

(32) The medicament according to (31) above, which is a regulator for the regeneration, growth or differentiation of a cell.

(32a) A regulator for the regeneration, growth or differentiation of a cell comprising a protein comprising the same or substantially the same amino acid sequence as the amino acid sequence represented by SEQ ID NO: 1, which comprises cholecystokinin or its analogue.

(33) The regulator according to (32) or (32a) above, which is capable of regulating the differentiation of a stem cell.

(34) The regulator according to (32) or (32a) above, which is capable of regulating the differentiation of a mesenchymal stem cell.

(35) The regulator according to (32) or (32a) above, which is capable of regulating the differentiation into a fat cell.

(36) A cell, which is obtained by regulating the differentiation with the regulator obtained by the method according to (16) above or with the regulator according to (31) above.

(37) A method of regulating the regeneration, growth or differentiation of a cell, which comprises using the regulator according to (16) or (32a) above.

(37a) A method of regenerating an organ, which comprises using the method according to (37) above.

(38) A method of treating a disease associated with the regeneration, growth or differentiation of a cell, which comprises administering the regulator according to (16) or (32a) above.

(39) The method according to (38) above, wherein the disease is obesity, diabetes mellitus, an immune disorder, a liver disorder, an alimentary disorder, or tissue damages in plastic surgery.

(40) A method of screening a compound capable of regulating the regeneration, growth or differentiation of a cell, which comprises using a protein comprising the amino acid sequence represented by SEQ ID NO: 1, or its partial peptide.

(41) A kit for screening a compound capable of regulating the regeneration, growth or differentiation of a cell, which comprises a protein comprising the amino acid sequence represented by SEQ ID NO: 1, or its partial- peptide.

(42) A marker for a somatic stem cell, which comprises a compound capable of binding to a protein comprising the same or substantially the same amino acid sequence as the amino acid sequence represented by SEQ ID NO: 1.

(43) The marker according to (42) above, wherein a somatic stem cell is labeled with the compound, whereby the marker is usable for discriminating, selecting or condensing the cell.

(44) The marker according to (42) above, which is capable of labeling the mesenchymal stem cell derived from bone marrow.

(45) A diagnostic agent for diagnosis of a disease associated with the regeneration, growth or differentiation of a cell, which comprises an antibody to the gene product of the gene identified by the method according to (1) above.

(46) A diagnostic kit for diagnosis of a disease associated with the regeneration, growth or differentiation of a cell, which comprises an antibody to the gene product of the gene identified by the method according to (1) above.

(47) A method for diagnosis of a disease associated with the regeneration, growth or differentiation of a cell, which comprises determining the expression level of a gene associated with the regeneration, growth or differentiation of the cell, using the diagnostic agent according to (45) above or the diagnostic kit according to (46) above.

(48) A diagnostic agent for diagnosis of a disease associated with the regeneration, growth or differentiation of a cell, which comprises a polynucleotide encoding the gene product of the gene identified by the method according to (1) above.

(49) A diagnostic kit for diagnosis of a disease associated with the regeneration, growth or differentiation of a cell, which comprises a polynucleotide encoding the gene product of the gene identified by the method according to (1) above.

(50) A method for diagnosis of a disease associated with the regeneration, growth or differentiation of a cell, which comprises quantifying mRNA translated from a gene associated with the regeneration, growth or differentiation of a cell, using the diagnostic agent according to (48) above or the diagnostic kit according to (49) above.
(51) A method of treatment by compensating for the lost function due to a damage caused by transplantation or a disease, which comprises using the cell according to (36) above.
(52) A method of screening a medicament candidate compound, which comprises using the cell according to (36) above.
(53) A method of confirming the safety of a medicament candidate compound, which comprises using the cell according to (36) above.
(54) A method of differentiation-inducing a human mesenchymal stem cell into an osteoblast cell, which comprises culturing the human mesenchymal stem cell on a serum-free medium.
(55) The method according to (54) above, wherein oncostatin M is added to the serum-free medium.
(56) A cell differentiation inducer for a serum free medium, which comprises oncostatin M.
(57) The cell differentiation inducer according to (56) above, which differentiates and induces a human mesenchymal stem cell into an osteoblast cell.

According to the methods of the present invention for screening genes associated with cell regeneration, growth or differentiation, the genes associated with cell regeneration, growth or differentiation can be efficiently identified in cells having the regeneration, differentiation or growth capability in a few steps. Further according to the methods of the present invention for screening regulators for cell regeneration, growth or differentiation, the regulators for cell regeneration, growth or differentiation can be obtained more efficiently than in conventional methods.

The regulators for cell regeneration, growth or differentiation identified by these methods enable to regulate the regeneration, growth or differentiation of cells and are thus useful as agents for the prevention and/or treatment of disorders or injuries, for example, diseases associated with cell regeneration, growth or differentiation, for example, disorders or injuries such as central system disorders, neurological disorders, inflammatory disorders, circulatory disorders, cancer, obesity, diabetes mellitus, immune disorders, liver/gallbladder disorders, alimentary disorders, heat burn, fracture, osteoarthritis, periodontal disease, alopecia, etc.

In addition, according to the diagnostic agents, diagnostic kits and methods for diagnosis of the present invention for diagnosis of diseases associated with cell regeneration, growth or differentiation, it is advantageous in that diseases associated with cell regeneration, growth or differentiation can be diagnosed in a simple manner.

## BRIEF DESCRIPTION OF THE DRAWINGS

FIG. 1 shows the results of experiments to confirm the function of differentiation-inducing cholecystokinin into adipocytes.
FIG 2 shows the calcium level accumulated on Day 10 of the incubation.

## BEST MODE FOR CARRYING OUT THE INVENTION

Hereinafter the present invention is described in detail.

(Method of Screening Genes Associated with Cell Regeneration, Growth or Differentiation)

In the methods of screening the genes associated with cell regeneration, growth or differentiation, the present invention is characterized in identifying the genes associated with cell regeneration, growth or differentiation by quantitatively analyzing the expression levels of multiple genes collectively in cells having the regeneration, differentiation or growth capability. Since such methods of quantitatively analyzing the expression levels of multiple genes exhaustively in cells having the regeneration, differentiation or growth capability have been yet unknown, the methods of the present invention are epoch-making methods for analyzing the functions of multiple genes in cells having the regeneration, differentiation or growth capability. In fact, a novel function of the cholecystokinin receptor has been elucidated by using the gene analysis methods of the present invention, which have proved the effectiveness of the methods of the present invention. In accordance with a preferred embodiment of the present invention, the screening method comprises (1) the step of quantitatively analyzing the expression levels of multiple genes collectively in a cell having a regeneration, differentiation or growth capability thereby to identify one or more genes that show higher expression levels in the cell or are specific in expression as compared to the other cells, and (2) the step of bringing at least one candidate substance capable of regulating the function of the identified genes or their gene products in contact with the cell and comparing changes before and after the contact. Herein, the term "show higher expression levels" is used to mean that expression levels of genes expressed in a cell are relatively high as compared to those of the other genes. For example, when compared in the mRNA level, it means the expression level that belongs to the top 20%, preferably the top 10%. The term "specific in expression as compared to the other cells" is used to mean that the expression is different to such an extent that there is a physiological significance, when compared to the expression of a gene in a cell different from the target cell under analysis, and refers to, for instance, a case in which the expression level is higher than that of mature cells (e.g., higher by 20% or more, preferably higher by 30% or more and more preferably higher by 50% or more), a case in which the expression level is lower than that of mature cells (e.g., lower by 20% or more, preferably lower by 30% or more and more preferably lower by 50% or more), or a case in which the expression is enhanced or suppressed at a specific stage of cell differentiation.

In the present invention, the targeted gene is not particularly limited and is selected from, e.g., a group of genes selected from the G protein-coupled receptor gene family; the nuclear receptor gene family; the tyrosine kinase-type receptor gene family; the transcription factor; gene families related to any one of protein kinases, protein phosphatases, proteases, ATPases, GTPases, DNA-bound proteins, cell adhesion factors and their receptors, ion channels, transporters, extracellular matrix components, intracellular cytoskeleton components, cell growth factors, cytokines, neurotrophic factors, physiologically active peptides, hormones, oxidases or reductases, hydrolases, hydroxylases, methylases or demethylases, transferases, ribosome-constituting proteins and histocompatible antigen proteins; and a group of genes which act directly or indirectly on the above genes to regulate their activities (preferably a group of genes selected from the G protein-coupled receptor gene family; the nuclear receptor gene family; and the protein kinase-type receptor gene family; etc.).

The multiple genes refer to two or more genes but have no particular upper limit so long as the number is practicable; normally it is 2 to several tens of thousands, preferably 2 to 1000, more preferably 10 to 800 and most preferably 10 to 300 of genes. Preferably, the multiple genes are a group of genes which share some common functions, e.g., a G protein-coupled receptor gene family; a nuclear receptor gene family; a protein kinase-type receptor gene family; etc. It is also preferred to quantitatively analyze the genes belonging to the gene group exhaustively without omission (preferably at least 50%, more preferably at least about 60%, still more preferably at least about 70%, still even more preferably at least about 80%, still yet even more preferably at least about 90%, much more preferably at least about 95% and most preferably at least about 99%, of the genes belonging to the gene group), more preferably, at least 70% of the genes belonging to the gene group; still more preferably, at least 90% of the genes belonging to the gene group; and particularly preferably, at least 95% of the genes belonging to the gene group; under the same conditions at a stretch (preferably concurrently).

More specifically, the gene expression analysis of the present invention is performed by bringing an mRNA sample, which may contain multiple target mRNAs, in contact with amplification reagents, each of which comprises one pair of primers corresponding to each target mRNA, at multiple reaction sites, preferably at the individual reaction sites of a reactor having multiple reaction sites to effect amplification, and determining the amounts of the amplification products formed. This method is explained below.

(mRNA Sample)

In a preferred embodiment of the present invention, the gene expression analysis is conducted by quantifying mRNAs from multiple target genes, which may be contained in an mRNA sample obtained from cells having the regeneration, differentiation or growth capability. Herein, the term "mRNA sample" is used to mean a sample which contains mRNAs and which is used for determining the types and levels of mRNAs contained in the sample.

More specifically, the mRNA sample targeted in the present invention is a sample used for analyzing the expression levels of specific genes in the sample, and are collected from the cells of, e.g., human or other mammals (for example, rats, mice, rabbits, sheep, swine, bovine, cats, dogs, monkeys, etc.), which have the regeneration, differentiation or growth capability. Such cells having the regeneration, differentiation or growth capability include embryonic stem cells, somatic stem cells (including mesenchymal stem cells derived from bone marrow, hematopoietic stem cells, stem cells from umbilical cord and umbilical cord blood, etc.), primary culture cells, cell lines, and the like. These cells are available from various tissues. Examples of such tissues include brain, various parts of the brain (e.g., olfactory bulb, amygdaloid nucleus, basal ganglia, hippocampus, thalamus, hypothalamus, subthalamic nucleus, cerebral cortex, medulla oblongata, cerebellum, occipital lobe, frontal lobe, temporal lobe, putamen, caudate nucleus, corpus callosum and substantia nigra), spinal cord, pituitary gland, stomach, pancreas, kidney, liver, gonad, thyroid, gall bladder, bone marrow, adrenal gland, skin, muscle, lung, digestive tract (e.g., large and small intestines), blood vessel, heart, thymus, spleen, submandibular, gland, peripheral blood, peripheral blood cell, prostate, testicle, testes, ovary, placenta, uterus, bone, joint, skeletal muscle and the like. The expression level of the target gene in the mRNA sample, which is obtained from cells having the regeneration, differentiation or growth capability, can be analyzed by quantifying the target gene contained in the sample.

In the present invention, by using the mRNA sample obtained from cells having the regeneration, differentiation or growth capability, it can be predicted that the genes which show higher expression levels in the cells or are specific in expression as compared to the other cells are associated with the regeneration, differentiation or growth in the cells. Whether the genes thus predicted are associated with regeneration, differentiation or growth or not can be determined by bringing at least one candidate substance capable of regulating the function of the identified genes or their gene products in contact with the cells and comparing the changes before and after the contact. As used herein, "at least one candidate substance capable of regulating the function of the identified genes or their gene products" includes, for example, peptides, proteins, non-peptide compounds, synthetic compounds, fermentation products, cell extracts, plant extracts, animal tissue extracts, plasma, etc. These compounds may be novel compounds or may be publicly known compounds.

In this comparison test, when the changes in cell regeneration, growth or differentiation a cell occurs to a physiologically significant extent after the candidate substance is contacted, the gene can be determined to be associated with the regeneration, growth or differentiation of the cell. As used herein, the physiologically significant extent refers to the case that, for example, when the candidate compound is contacted, the regeneration, growth or differentiation of the cell is promoted or suppressed by at least 10%, preferably at least 20% and more preferably at least 30%, as compared to the case where the candidate compound is not contacted.

Quantification of the expressed gene levels or computation of absolute values for the expressed gene levels can be performed according to the methods of quantifying the target mRNA, which will be described later.

(Reactor with Multiple Reaction Sites)

The screening method described above can be performed efficiently, using the reactor with multiple reaction sites.

No particular limit is imposed on the number of multiple reaction sites as long as there are two or more reaction sites. The number of reaction sites is normally 2 to several tens of thousands, preferably 2 to 1000, more preferably 10 to 800 and still more preferably 10 to 300.

According to the present invention, in order to amplify the target mRNAs which may be contained in an mRNA sample, the sample is reacted all at once with individual amplification reagents, each of which contains one pair of primers corresponding to its target mRNA. Consequently, the reactor having multiple reaction sites are preferably used in the present invention and reactions of the mRNA sample with individual amplification reagents are carried out at individual reaction sites. The construction and structure of the reactor used in the present invention are not particularly limited as far as the reactor has two or more reaction sites where the mRNA sample is reacted with individual amplification reagents all at once. Preferably, the reactor used in the present invention includes, for example, a plate having multiple wells, a reactor equipped with multiple glass slides, a reactor equipped with multiple test tubes, etc. Taking into account the testing space, operability, etc., the plate having multiple wells can be preferably used. These plates can be chosen depending upon the number of primer pairs used, and commercially available 96-well and 384-well plates are preferably used. However, the reactor equipped with a desired number of reaction sites corresponding to the number of primer pairs can be used as well. The method of the present invention can also be performed by using two or more 96-well or 384-well plates commercially available.

(Amplification Reagent)

Each of the amplification reagents used in the present invention comprises, for example, one pair of primers corresponding to the target mRNA, a probe corresponding to the target mRNA, a DNA polymerase, a buffer and the like.

Herein, the term "one pair of primers corresponding to the target mRNA" used in the present invention refers to one pair of primers consisting of a first primer complementary or substantially complementary to one strand of the exon region of the target gene sequence encoding the target mRNA and a second primer complementary or substantially complementary to the other strand of the exon region of the target gene sequence. In the present invention, the presence or absence of mRNA transcribed from the target genes in an mRNA sample and its transcription level can be detected all at once by using two or more pairs of these primer pairs. Therefore, the greater the number of primer pairs used, the more efficient the methods of the present invention for quantitative determination.

According to the method of the present invention for quantitative determination, the number of the primer pairs group used is not particularly limited because the group may be adjusted depending on the number of target mRNAs, and the group may be composed of, e.g., 10 to 800 pairs of primers. In another embodiment of the present invention, the primer pairs group used is composed of 10 to 300 pairs of primers. When the number of primer pairs is large, different sets of amplification reagents are previously assigned to multiple plates (e.g., 2 to 10 plates) and the reaction for quantitative determination may be carried out dividedly in plural times.

In a preferred embodiment of the present invention, the target gene is a gene selected from the G protein-coupled receptor gene family, the nuclear receptor gene family and the protein kinase-type receptor gene family. Thus, the target mRNA is mRNA in the gene group belonging to the G protein-coupled receptor gene family, the nuclear receptor gene family and the protein kinase-type receptor gene family.

In this case, the amplification reagents each comprising one pair of primers corresponding to mRNA in the target gene group described above are brought in contact with an mRNA sample at the respective reaction sites of the reactor to effect amplification, and the mRNA amplification products are quantitatively determined, whereby the expression levels of the target genes in the groups described above which are contained in the mRNA sample can be quantified.

In a more preferred embodiment of the present invention, all pairs of primers corresponding to the mRNAs of genes belonging to all known G protein-coupled receptor, nuclear receptor and protein kinase-type receptor gene families are prepared and amplification reagents comprising their respective pairs of primers are set at the respective reaction sites; then it can be determined which mRNA is produced from which gene and how much is produced in the mRNA sample in a single assay operation. It is of course possible, when necessary, to assign different sets of amplification reagents to multiple plates and perform the quantitative assay reaction dividedly in plural times.

The following are currently known as the G protein-coupled receptors (or genes):

(1) Acetylcholine receptors: $M_1$; $M_2$; $M_3$; $M_4$; $M_5$
(2) Adenosine receptors: $A_1$; $A_{2A}$; $A_{2B}$; $A_3$
(3) Adrenoceptors: α1A; α1B; α1D; α2A; α2B; α2C; β1; β2; β3
(4) Angiotensin receptors: AT1; AT2
(5) Bombesin receptors: BB1; BB2; BB3
(6) Bradykinin receptors: $B_1$; $B_2$
(7) Calcitonin-amylin-CGRP and adrenomedullin receptors:
(8) Cannabinoid receptors: CB1; CB2
(9) Chemokine receptors: CCR1; CCR2; CCR3; CCR4; CCR5; CCR6; CCR7; CCR8; CCR9; CCR10; CXCR1; CXCR2; CXCR3; CXCR4; CXCR5; CX3CR1; XCR1
(10) Cheniotactic peptide receptors: C3a; C5a; fMLP
(11) Cholecystokinin and gastrin receptors: $CCK_1$; $CCK_2$
(12) Corticotropin-releasing factor receptors: $CRF_1$; $CRF_2$
(13) Dopamine receptors: D1; D2; D3; D4; D5
(14) Endothelin receptors: $ET_A$; $ET_B$
(15) Galanin receptors: GAL1; GAL2; GAL3
(16) Glutamate receptors: $mglu_1$; $mglu_2$; $mglu_3$; $mglu_4$; $mglu_5$; $mglu_6$; $mglu_7$; $mglu_8$
(17) Glycoprotein hormonereceptors: FSH; LSH; TSH
(18) Histamine receptors: $H_1$; $H_2$; $H_3$; $H_4$
(19) 5-HT receptors: 5-$HT_{1A}$; 5-$HT_{1B}$; 5-$HT_{1D}$; 5-$ht_{1B}$; 5-$ht_{1F}$;5-$HT_{2A}$; 5-$HT_{2F}$; 5-$HT_{2C}$; 5-$HT_3$; 5-$HT_4$; 5-$ht_{5A}$; 5-$ht_{5B}$; 5-$HT_6$; 5-$HT_7$
(20) Leukotriene receptors: BLT; $CysLT_1$; $CysLT_2$
(21) Lysophospholipid receptors: edg1; edg2; edg3; edg4
(22) Melanocortin receptors: $MC_1$; $MC_2$; $MC_3$; $MC_4$; $MC_5$
(23) Melatonin receptors: $MT_1$; $MT_2$; $MT_3$
(24) Neuropeptide Y receptors: $Y_1$; $Y_2$; $Y_4$; $Y_5$; $Y_6$
(25) Neurotensin receptors: NTS1; NTS2
(26) Opioid receptors: DOP; KOP; MOP; NOP
(27)P2Y receptors: $P2Y_1$; $P2Y_2$; $P2Y_4$; $P2Y_6$; $P2Y_{11}$; $P2Y_{12}$
(28) Prostanoid receptors: DP; FP; IP; TP; $EP_I$; $EP_2$; $EP_3$; $EP_4$
(29) Protease-activated receptors: PAR1; PAR2; PAR3; PAR4
(30) Somatotatin receptors: $sst_1$; $sst_2$; $sst_3$; $sst_4$; $sst_5$
(31) Tachykinin receptors: $NK_1$; $NK_2$; $NK_3$
(32) Thyrotropin-releasing hormone receptors: $TRH_1$; $TRH_2$
(33) Urotensin-II receptors:
(34) Vasoactive intestinal peptide and pituitary adenylate cyclase activating peptide receptors: $VPAC_1$; $VPAC_2$; $PAC_1$
(35) Vasopressin and oxytocin receptors: $V_{1a}$; $V_{1b}$; $V_2$; OT

In addition, the following are currently known as the genes belonging to the families such as tyrosine kinase-type receptors, ion channel genes, etc.

(36) Ion channels: $Na^+$ channels (Type I; Type II/Type IIA; Type III; SCL11/NaG; PN1; NaCh6; NaDRG; SkM1/μl, SkM2), $K^+$ channels (Kv; EAG; KQT; IRK; ROMK; GIRK; $K_{ATP}$, etc.), $Ca^{2+}$ channels (α1G; α1E; α1S; α1C; α1D; α1B; α1A; IP3; ryanodine receptor receptors, etc.), $Cl^-$ channels ($GABA_A$; GABAc; glycine receptors; C1C0; C1C1; CFTR, etc.), non-selective cation channels (nAChR; 5-$HT_3$; NMDA; AMPA; $P_{2X}$ATP; CNG, etc.), etc.

(37) Tyrosine kinase receptors: insulin receptors; EGF receptors, etc.

Furthermore, the following are known as nuclear receptors; for example:

(38) Peroxisome proliferator-activated receptors: PPAR-α; PPAR-β; PPAR-γ, etc.

The pair of primers for amplifying the target mRNA can easily be designed by a person having ordinary skill in the art, based on the sequence of its target GPCR gene (see, e.g., Genome Res. 1996 Oct., 6(10): 986-94).

Next, probes corresponding to the target mRNAs used in the present invention can easily be designed by a person having ordinary skill in the art, based on the target sequence (see, e.g., Genome Res. Oct. 6, 1996(10): 986-94).

Appropriate oligonucleotide probes used in the present invention have a length of preferably about 15 to about 50 nucleotides, and more preferably about 25 to about 35 nucleotides. The oligonucleotide probes can be labeled by incorporating a detectable chemical substance or the like by biochemical, immunochemical or chemical means. Useful labels include radioactive isotopes such as $^{32}P$, etc., fluorescent substances such as fluorescamine, fluorescein isothiocyanate, etc., luminescent substances such as luminol, luciferin, etc., enzymes such as β-galactosidase, peroxidase, alkaliphosphatase, etc., biotin, antibodies, and the like.

The DNA polymerase used in the present invention includes, for example, heat-resistant DNA polymerase having the reverse transcription and 5'→3' exonuclease activities, such as rTth DNA polymerase, and the like.

In the present invention, buffers publicly known or commercially available can be used (buffers manufactured by PE Biosystems; see, e.g., Genome Res. Oct. 6, 1996(10): 986-94).

(Amplification of mRNA)

According to the analysis method of the present invention, the target mRNA, which may be contained in an mRNA sample, is amplified using an amplification reagent comprising a pair of primers for amplifying the target mRNA. In a preferred embodiment of the present invention, the target mRNA is amplified by the conventional polymerase chain reaction (PCR) (see U. S. Patent Nos. 4,683,195, 4,683,202 and 4,965,188, etc.).

The amplification of mRNA can be effected by reverse transcription of the target mRNA using, e.g., a virus reverse transcriptase, followed by amplifying the resulting cDNA. In a still more preferred embodiment, mRNA is amplified by the reverse transcription-polymerase chain reaction (RT-PCR) (see U.S. Patent Nos. 5,310,652, 5,322,770, 5,561,058, 5,641,864, 5,693,517, etc.).

In the present invention, a variety of mRNA amplification techniques other than the polymerase chain reactions described above can also be used. Such amplification techniques include, for example, the strand-displacement amplification (see U.S. Patent No. 5,455,166, etc.), the transcription-based amplification system (TAS) (see U.S. Patent Nos. 5,437,990, 5,409,818, 5,399,491, etc.) and the self-sustained sequence replication (3SR) (see WO 92/08800, etc.).

The reaction conditions for these amplification reactions can be easily designed by a person having ordinary skill in the art, depending upon the types of reagents used and the like.

(Method of Quantitative Determination of Target mRNA)

Next, according to a preferred method of the present invention, the amount of the aforesaid mRNA amplification product produced is quantitatively determined. Preferably, the amplification product is quantitatively determined by the methods using probes. In a preferred embodiment, the amplification product is quantitatively determined by the methods using probes labeled with fluorescent substances.

In a more preferred embodiment of the present invention, the target mRNA is quantitatively determined using the "TaqMan method" or "5' nuclease assay method" (Proceedings of the National Academy of Sciences U.S.A., Vol. 88, pp. 7276-7280 (1991); U.S. Patent Nos. 5,210,015, 5,487,972, 5,804,375, etc.). In the TaqMan assay method, a probe labeled at the 5' end is used. The 3' end of this probe is further modified to prevent the probe from acting as a primer for DNA synthesis. The modification includes addition of a phosphate group, a fluorescent substance, etc. to the end. The target mRNA is amplified using a DNA polymerase having the 5'→3' exonuclease activity such as Tth DNA polymerase. The probe, which hybridizes to the target mRNA downstream from the primer, is degraded by the 5'→3' exonuclease activity of DNA polymerase during the amplification reaction. Whenever a new target region is amplified, the probe is degraded and the label substance is released. By quantitatively assaying this released label substance, the amount of target mRNA can be determined indirectly.

For detecting the released label substance quantitatively, publicly known methods are used. According to a preferred method, the probe described above is labeled at the 5' and 3' ends with two fluorescent substances, one of which can quench the fluorescence of the other substance. This probe quenches fluorescence through interaction of the two fluorescent substances while the probe is hybridized to the template DNA, but emits fluorescence when the probe is degraded by the 5'→3' exonuclease activity of the DNA polymerase. As the amplification proceeds, the fluorescence increases and this increase is monitored.

A sample containing the target mRNA is quantitatively assayed based on a "standard curve" which is previously prepared by using and amplifying a sample containing a known quantity of target mRNA. The standard curve is used to calculate the number of input copies, which are derived from signals emitted during the amplification. Accordingly, an unknown number of copies of the target sequence in a sample is estimated by calculating the number of copies which has been determined in advance to emit the signal equivalent to what is previously observed using the standard curve described above. Next, the concentration of the target sequence in the sample can be calculated from the number of input copies determined before the reaction and the size of the sample (see Japanese Patent KOKAI No. 2001-204483, etc.).

An internal standard can be used to eliminate experimental errors. Quantitative assay methods based on amplification using internal standards are disclosed in U.S. Patent Nos. 5,219,717 and 5,476,774, etc. Various methods are known for improving the specificity of the amplification reaction and described, for example in European Patent Application No. 0 866,071, etc.

(Method of Screening Regulator for Cell Regeneration, Growth or Differentiation)

In another embodiment of the present invention, there is provided a method of screening a regulator for cell regeneration, growth or differentiation (or a compound for regulating cell growth or differentiation), which comprises the step of quantitatively analyzing expression levels of multiple genes collectively in a cell having a regeneration, differentiation or growth capability to thereby identify one or more genes that show higher expression levels in the cell or are specific in expression as compared to the other cells; and, the step of bringing at least one candidate substance which can regulate the function of the identified genes or their gene products in contact with the cell and comparing changes before and after the contact. In the above comparison test in accordance with such a method, when a difference in cell regeneration, growth or differentiation is observed to a physiologically significant extent before or after a certain candidate substance is brought in contact with the cell, the candidate substance can be determined to be associated with cell regeneration, differentiation or growth. As used herein, "at least one candidate substance which can regulate the function of the genes or their gene products" include, for example, peptides, proteins, non-peptide compounds, synthetic compounds, fermentation products, cell extracts, plant extracts, animal tissue extracts, plasma, etc. These compounds may be novel compounds or may be publicly known compounds. The "physiologically significant extent" is used to mean the case that, for example, when the candidate compound is contacted, the cell regeneration, growth or differentiation is promoted or suppressed by at least 10%, preferably at least 20% and more preferably at least 30%, as compared to the case where the candidate compound is not contacted. As described above, the substance associated with cell regeneration, differentiation or growth can be used as the regulator for cell regeneration, differentiation or growth. As used herein, the "regulator for regeneration, differentiation or growth of a cell" includes a substance that promotes cell regeneration, differentiation or growth and a substance that suppresses cell regeneration, differentiation or growth.

(Medicament Comprising Substance Obtained by Screening Methods Above, etc.)

Next, the present invention also relates to medicaments comprising agonists, antagonists, inhibitors, activators or antibodies, to the gene products of the genes identified by the screening method according to (1) above, or polynucleotides (e.g., DNAs) encoding the gene products or antisense polynucleotides thereof As used herein, the "medicament" is used to mean that an individual compound itself is contained. Preferably, this medicament is the regulator for cell regeneration, growth or differentiation and using such a medicament, cell regeneration, growth or differentiation can be regulated. More specifically, where the expression of a particular gene or its gene product (e.g., a receptor protein) decreases in the body of a patient to cause abnormality in cell regeneration, growth or differentiation with which a particular gene or its gene product (e.g., a receptor protein) is associated, (1) the gene product (e.g., a receptor protein) is administered to the patient, whereby the level of the gene product is replenished, or (2) either (a) a DNA encoding the gene product is administered to the patient to induce the expression or (b) a DNA encoding the receptor protein of the present invention is inserted into the target cells to induce the expression and then the cells are transplanted into the patient, whereby the level of the gene product is increased in the body of the patient; thus, the action of the gene or gene product can be fully exhibited. Also, by inhibiting or activating the gene product of the gene identified by the screening method described in (1) above, the regeneration, growth or differentiation of the cell containing the gene product can be regulated.

Accordingly, the medicament of the present invention is effective for the prevention or treatment of disorders with which the regeneration, growth or differentiation of a cell containing the identified gene is associated and is therefore useful for the prevention or treatment of diseases or injuries, for example, central system disorders/neurological disorders (e.g., Alzheimer's disease, Parkinson's disease, ischemic neuropathy, etc.), inflammatory disorders (e.g., allergic diseases, asthma, rheumatism, degenerative joint disease, etc.), circulatory disorders (e.g., myocardial infarction, heart failure, hypertrophy, angina pectoris, arterial sclerosis, etc.), cancers (e.g., non-small-cell lung cancer, ovarian cancer, prostate cancer, gastric cancer, bladder cancer, breast cancer, cervical cancer, colon cancer, rectal cancer, etc.), overweight, diabetes mellitus, immune system disorders (e.g., autoimmune disease, atopic dermatitis, allergic diseases, immunodeficiency, asthma, rheumatoid arthritis, psoriasis, arterial sclerosis, diabetes mellitus, Alzheimer's disease, etc.), liver/gallbladder disorders (e.g., hepatic cirrhosis, hepatitis, hepatic insufficiency, cholestasis, calculus, etc.), alimentary disorders (e.g., ulcer, enteritis, indigestion, irritable bowel syndrome, ulcerative colitis, diarrhea, ileus, etc.), heat burn, fracture, osteoarthritis, periodontal disease, alopecia, etc.

The gene products (e.g., receptor proteins) of the gene identified in the present invention, agonists, antagonists, inhibitors, activators or antibodies, to the gene products, polynucleotides (e.g., DNAs) encoding the gene or their antisense polynucleotides can be used as the preventive and/or therapeutic agents described above, and in this case, they can be prepared into pharmaceutical preparations in a conventional manner.

For example, the medicament used in the present invention can be used orally, for example, in the form of tablets which may be sugar coated if necessary and desired, capsules, elixirs, microcapsules etc., or parenterally in the form of injectable preparations such as a sterile solution and a suspension in water or with other pharmaceutically acceptable liquid. These preparations can be manufactured by mixing the medicament of the present invention with a physiologically acceptable known carrier, a flavoring agent, an excipient, a vehicle, an antiseptic agent, a stabilizer, a binder, etc. in a unit dosage form required in a generally accepted manner that is applied to making pharmaceutical preparations. The effective component in the preparation is controlled in such a dose that an appropriate dose is obtained within the specified range given.

Additives miscible with tablets, capsules, etc. include a binder such as gelatin, corn starch, tragacanth and gum arabic, an excipient such as crystalline cellulose, a swelling agent such as corn starch, gelatin and alginic acid, a lubricant such as magnesium stearate, a sweetening agent such as sucrose, lactose and saccharin, and a flavoring agent such as peppermint, akamono oil and cherry. When the unit dosage is in the form of capsules, liquid carriers such as oils and fats may further be used together with the additives described above. A sterile composition for injection may be formulated by conventional procedures used to make pharmaceutical compositions, e.g., by dissolving or suspending the active ingredients in a vehicle such as water for injection with a naturally occurring vegetable oil such as sesame oil and coconut oil, etc. to prepare the pharmaceutical composition. Examples of an aqueous medium for injection include physiological saline and an isotonic solution containing glucose and other auxiliary agents (e.g., D-sorbitol, D-mannitol, sodium chloride, etc.) and may be used in combination with an appropriate dissolution aid such as an alcohol (e.g., ethanol), a polyalcohol (e.g., propylene glycol and polyethylene glycol), a nonionic surfactant (e.g., polysorbate 80™ and HCO-50), etc. Examples of the oily medium include sesame oil and soybean oil, which may also be used in combination with a dissolution aid such as benzyl benzoate and benzyl alcohol.

The preventive and/or therapeutic agents described above may further be formulated with a buffer (e.g., phosphate buffer, sodium acetate buffer, etc.), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus-prepared liquid for injection is normally filled in an appropriate ampoule.

Since the thus obtained pharmaceutical preparations is safe and low toxic, the preparation can be administered to human or other mammal (e.g., rats, mice, rabbits, sheep, swine, bovine, cats, dogs, monkeys, etc.).

The dose of the preventive and/or therapeutic agent of the present invention varies depending on subject to be administered, organs to be administered, conditions, routes for administration, etc.; in oral administration, e.g., for the patient with cancer, the dose is normally about 0.1 mg to about 100 mg, preferably about 1.0 to about 50 mg, and more preferably about 1.0 to about 20 mg per day (as 60 kg body weight). In parenteral administration, the single dose varies depending on subject to be administered, target organ, conditions, routes for administration, etc. but it is advantageous, e.g., for the patient with cancer, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

Where the DNA (hereinafter sometimes referred to as the DNA of the present invention) is used as the preventive and/or therapeutic agent described above, the DNA is administered alone; alternatively, the DNA is inserted into an appropriate vector such as retrovirus vector, adenovirus vector, adenovirus-associated virus vector, etc. and then administered to human or other warm-blooded animal in a conventional manner. The DNA may also be administered as intact DNA, or with pharmacologically acceptable carrier such as adjuvants to assist its uptake by gene gun or through a catheter such as a catheter with a hydrogel.

The dose of the DNA of the present invention varies depending on subject to be administered, organs to be administered, conditions, routes for administration, etc.; in oral administration, e.g., for the patient with cancer, the dose is normally about 0.1 mg to about 100 mg, preferably about 1.0 to about 50 mg, and more preferably about 1.0 to about 20 mg per day (as 60 kg body weight). In parenteral administration, the single dose varies depending on subject to be administered, target organ, conditions, routes for administration, etc. but it is advantageous, e.g., for the patient with cancer, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

The regulator of the present invention for cell regeneration, growth or differentiation can regulate the regeneration, growth or differentiation of cells such as embryonic stem cells, somatic stem cells, mesenchymal stem cells, splenocytes, nerve cells, glial cells, pancreatic (α, β, δ) cells, chondrocytes, osteocytes, bone marrow cells, mesangial cells, epidermic cells, epithelial cells, endothelial cells, fibroblasts, fibrocytes, muscle (skeletal muscle/cardiac muscle) cells, fat cells, immune cells, vascular endothelial cells, endothelial progenitor cells, hematopoietic cells, etc. The regeneration, growth or differentiation of such cells can be regulated in vivo by administration to the patient as described above, or in vitro. Where the regeneration, growth or differentiation of cells are performed in vitro, the regulation can be effected by culturing target cells in the presence of the regulator of the present invention for cell regeneration, growth or differentiation. Culture conditions (medium, temperature, time, the amount of the regulator used, etc.) used herein can be appropriately determined depending upon kind of target cells, kind of regulators for cell regeneration, growth or differentiation, etc. The methods and conditions for culture can be appropriately determined by referring to, e.g., journals such as Molecular Medicine Vol. 40, 144-151 (2003); Molecular Medicine Vol. 40, 265-270 (2003); Nat. Med. 5, 975-977 (1999); Embryonic Stem Cell Differentiation in vitro (M. V. Wiles, Meth. Enzymol. 225:900 (1993); Meth. Cell Biol. 75: 173 (1997); Large Scale Mammalian Cell Culture Curr. Opin. Biotechnol. 8:148 (1997), etc. For example, target cells are incubated at 20-45°C (preferably 37°C) in the presence or absence of feeder cells for 1-20 days in a $CO_2$ incubator in the presence of the regulator of the present invention for cell regeneration, growth or differentiation (e.g., cholecystokinin later described). When a factor associated with regeneration, growth or differentiation of the target cells is known, the incubation is carried out in the presence of such a factor. As factors involved in the growth of neurons there are known, for example, NGF, GDNF, BDNF, etc. and as blood cell growth factors there are known, for example, IL1, IL3, IL6, SCF, GM-SCF, EPO, etc. The present invention also includes cells acquired by regulating differentiation as above. The regeneration, growth or differentiation of cells can be confirmed by FACS analysis or observation with electron microscope. These cells thus obtained can also be used in the treatment for restoring the damages due to transplantation or the lost function. In addition, the cells thus obtained can be used for screening of drug candidate compounds or for safety confirmation of drug candidate compounds.

Also, the regulator of the present invention for cell regeneration, differentiation or growth can promote the regeneration, growth or differentiation of cells and hence can regenerate tissues or organs. The organs which can be regenerated as described above include brain, any of brain regions, cornea, spinal cord, hypophysis, stomach, pancreas, kidney, liver, gonad, thyroid, gall-bladder, bone marrow, adrenal gland, skin, muscle, lung, digestive tract, blood vessel, heart, thymus, submandibular gland, peripheral blood, peripheral blood cell, prostate, testicle, testis, ovary, placenta, uterus, tooth, thymus, bone, joint, skeletal muscle, etc. Where tissues or organs are regenerated using the regulator of the present invention for cell regeneration, differentiation or growth, the regulator prepared into a pharmaceutical preparation as described above is administered to the patient in such a manner as described above. Tissue or organ regeneration can also be achieved by known transplantation therapy. Such known techniques are described in journals such as Molecular Medicine Vol. 40, 2-8 (2003); Nat. Med. 5, 975-977 (1999); J. Thorac. Cardiovasc. Surg. 113, 10-18 (1997) ; Nat. Med. 7, 430-436 (2001); Nature 410, 701-705 (2001); J. Neurosci. Res. 561, 364-370 (2000); Stroke 32, 1005-1011 (2001); Science 288, 1660-1663 (2000), etc.

(Cholecystokinin Receptor, etc.)

Of the receptor protein comprising the same or substantially the same amino acid sequence as the amino acid sequence represented by SEQ ID NO: 1 obtained as a result of using the gene expression analysis method of the present invention described above, new applications are explained below.

In the present invention, the cholecystokinin receptor gene could be identified as the gene associated with cell regeneration, differentiation or growth by using the screening method described above (see EXAMPLES for the details). In this case, the cholecystokinin receptor protein refers to a receptor protein comprising the same or substantially the same amino acid sequence as the amino acid sequence represented by SEQ ID NO: 1 (hereinafter also referred to as "the receptor protein of the present invention"). Meanwhile, the protein having the amino acid sequence represented by SEQ ID NO: 1 is human cholecystokinin receptor, which has been registered in GenBank as Accession No. AAA35659 (SEQ ID NO: 1). Also, the base sequence of the gene encoding human cholecystokinin receptor has been registered in GenBank as Accession No. L13605 (SEQ ID NO: 2).

The receptor protein of the present invention may be any protein derived from any cells [e.g., retina cells, splenocytes, nerve cells, glial cells, β cells of pancreas, bone marrow cells, mesangial cells, Langerhans' cells, epidermic cells, epithelial cells, endothelial cells, fibroblasts, fibrocytes, myocytes, fat cells, immune cells (e.g., macrophage, T cells, β cells, natural killer cells, mast cells, neutrophil, basophil, eosinophil, monocyte, leukocyte), megakaryocyte, synovial cells, chondrocytes, bone cells, osteoblasts, osteoclasts, mammary gland cells, hepatocytes or interstitial cells, the corresponding precursor cells, stem cells, cancer cells (e.g., breast cancer cell lines (GI-101), colon cancer cell line (CX-1, GI-112), lung cancer cell line (LX-1, GI-117), ovarian cancer cell line (GI-102), prostate cancer cell line, etc.)

etc.], or any tissues where such cells are present, e.g., brain or any region of the brain (e.g., olfactory bulb, amygdaloid nucleus, basal ganglia, hippocampus, thalamus, hypothalamus, subthalamic nucleus, cerebral cortex, medulla oblongata, cerebellum, occipital pole, frontal lobe, temporal lobe, putamen, caudate nucleus, corpus callosum, substantia nigra), spinal cord, hypophysis, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, peripheral blood cells, prostate, testicle, testis, ovary, placenta, uterus, bone, joint, skeletal muscle, etc., or from blood cells or cultured cells thereof (e.g., MEL, M1, CTLL-2, HT-2, WEHI-3, HL-60, JOSK-1, K562, ML-1, MOLT-3, MOLT-4, MOLT-10, CCRF-CEM, TALL-1, Jurkat, CCRT-HSB-2, KE-37, SKW-3, HUT-78, HUT-102, H9,U937, THP-1, HEL, JK-1, CMK, KO-812, MEG-O1, etc.) from human and other mammals (e.g., guinea pigs, rats, mice, rabbits, swine, sheep, bovine, monkeys, etc.). The receptor protein may also be a synthetic protein.

As used herein, the term "substantially the same amino acid sequence" refers to an amino acid sequence having at least about 50% homology, preferably at least about 60% homology, more preferably at least about 70% homology, still more preferably at least about 80% homology, still yet more preferably at least about 90% homology, and most preferably at least about 95% homology, to amino acid sequences to be compared.

As the protein comprising substantially the same amino acid sequence as the amino acid sequence represented by SEQ ID NO: 9, preferred is, e.g., a protein comprising substantially the same amino acid sequence as the amino acid sequecnse represented by SEQ ID NO: 1 and having an activity substantially equivalent to the protein comprising the amino acid sequence represented by SEQ ID NO: 1, etc.

The substantially equivalent activities include, for example, cell regeneration, growth or differentiation activities, ligand binding activities, signal transduction activities, or the like. The substantially equivalent is used to mean that the nature of the activity is equivalent in terms of quality. Thus, the activity such as cell regeneration, growth or differentiation activities, ligand binding activities, signal transduction activities, or the like is preferably equivalent (e.g., about 0.01 to 100 times, preferably about 0.5 to 20 times, more preferably 0.5 to 2 times), but differences in quantitative factors such as a level of these activities, a molecular weight of the protein, etc. may be present and allowable.

The activities such as the cell regeneration, growth or differentiation activities, ligand binding activities, signal transduction actions or the like can be determined according to publicly known methods with some modifications thereof. For example, the activities can be assayed by the methods for determining ligands or the screening methods, which will be later described.

Also, proteins comprising the following amino acid sequences are used as the receptor proteins: (1) amino acid sequences wherein at least 1 or 2 amino acids (preferably approximately 1 to 30 amino acids, more preferably approximately 1 to 10 amino acids, most preferably 1 to several (1 to 5) amino acids) are deleted of the amino acid sequence represented by SEQ ID NO: 1; (2) amino acid sequences wherein at least 1 or 2 amino acids (preferably approximately 1 to 30 amino acids, more preferably approximately 1 to 10 amino acids, and most preferably 1 to several (1 to 5) amino acids) are added to the amino acid sequence represented by SEQ ID NO: 1; (3) amino acid sequences wherein at least 1 or 2 amino acids (preferably approximately 1 to 30 amino acids, more preferably approximately 1 to 10 amino acids, and most preferably 1 to several (1 to 5) amino acids) in the amino acid sequence represented by SEQ ID NO: 1 are substituted by other amino acids; or (4) amino acid sequences which are combination of these; and the like.

As used herein, the receptor protein is represented in accordance with the conventional way of describing peptides; the N-terminus (amino terminus) is at the left hand and the C-terminus (carboxyl terminus) is at the right hand. In the receptor protein of the present invention, the C-terminus may be in any form of a carboxyl group (-COOH), carboxylate ($-COO^-$), an amide ($-CONH_2$) or an ester (-COOR).

Examples ofR in the ester include a $C_{1-6}$ alkyl group such as methyl, ethyl, n-propyl, isopropyl or n-butyl, etc.; a $C_{3-8}$ cycloalkyl group such as cyclopentyl, cyclohexyl, etc.; a $C_{6-12}$ aryl group such as phenyl, $\alpha$-naphthyl, etc.; a $C_{7-14}$ aralkyl group such as a phenyl-$C_{1-2}$-alkyl group, e.g., benzyl, phenethyl, etc., or an $\alpha$-naphthyl-$C_{1-2}$-alkyl group such as $\alpha$-naphthylmethyl, etc.; and the like. In addition, pivaloyloxymethyl or the like, which is used widely as an ester for oral administration, may also be used.

Where the receptor protein of the present invention contains a carboxyl group (or a carboxylate) at a position other than the C-terminus, it may be amidated or esterified and such an amide or ester is also included within the receptor protein of the present invention. The ester group may be the same group as that described with respect to the C-terminus described above.

Furthermore, examples of the receptor protein of the present invention include variants of the above proteins, wherein the amino group at the N-terminal methionine residue of the protein supra is protected with a protecting group (for example, a $C_{1-6}$ acyl group such as a $C_{2-6}$ alkanoyl group, e.g., formyl group, acetyl group, etc.); those wherein the N-terminal region is cleaved in vivo and the glutamyl group thus formed is pyroglutaminated; those wherein a substituent (e.g., -OH, -SH, amino group, imidazole group, indole group, guanidino group, etc.) on the side chain of an amino acid in the molecule is protected with a suitable protecting group (e.g., a $C_{1-6}$ acyl group such as a $C_{2-6}$ alkanoyl group, e.g., formyl group, acetyl group, etc.), or conjugated proteins such as so-called glycoproteins having sugar chains bound thereto.

Specific examples of the receptor protein of the present invention include human-derived cholecystokinin receptor protein consisting of the amino acid sequence represented by SEQ ID NO: 1.

The receptor protein of the present invention or its salts can be manufactured from the human or mammalian cells or tissues described above by publicly known methods for purification of receptor proteins, or by culturing transformants bearing DNA encoding the receptor protein of the present invention later described. Alternatively, the receptor protein or its salts can also be manufactured by protein synthesis method or its modifications later described.

Where they are manufactured from human or mammalian tissues or cells, human or mammalian tissues or cells are homogenized, then extracted with an acid or the like, and the extract is isolated and purified by a combination of chromatography techniques such as reverse phase chromatography, ion exchange chromatography, and the like.

(Partial Peptide of Receptor Protein)

As a partial peptide of the present invention (hereinafter sometimes referred to as the "partial peptide"), any partial peptide can be used so long as it is a peptide having a part of the amino acid sequence of the receptor protein of the present invention described above. For example, among receptor protein molecules of the present invention, those having a site exposed to the outside of a cell membrane and having substantially the same receptor binding activity as the receptor protein of the present invention can be used.

Specifically, the partial peptide of the receptor protein of the present invention having the amino acid sequence represented by SEQ ID NO: 1 is a peptide containing the parts analyzed to be extracellular domains (hydrophilic domains) in the hydrophobic plotting analysis. A peptide containing the hydrophobic domain in part can be used as well. In addition, the peptide may contain each domain separately or plural domains together.

In the partial peptides of the present invention, preferred are peptides having at least 20, preferably at least 50, and more preferably at least 100 amino acids, in the amino acid sequence which constitutes the receptor protein of the present invention described above.

The term substantially the same amino acid sequence refers to an amino acid sequence having at least about 50% homology, preferably at least about 60% homology, more preferably at least about 70% homology, still more preferably at least about 80% homology, still even more preferably at least about 90% homology and most preferably at least about 95% homology, to these amino acid sequences.

Herein, "substantially equivalent receptor binding activity" has the same significance as above. "Substantially equivalent receptor binding activity" is assayed in the same way as described above.

In the amino acid sequence described above, the partial peptide of the present invention may contain amino acid sequences, of which at least 1 or 2 amino acids (preferably approximately 1 to 10 amino acids, more preferably 1 to several (1 to 5) amino acids) may be deleted; to which at least 1 or 2 amino acids (preferably approximately 1 to 20 amino acids, more preferably approximately 1 to 10 amino acids, and most preferably 1 to several (1 to 5) amino acids) may be added; or, in which at least 1 or 2 amino acids (preferably approximately 1 to 10 amino acids, more preferably 1 to several and most preferably approximately 1 to 5 amino acids) may be substituted with other amino acids.

In the partial peptide of the present invention, the C-terminus may be in any form of a carboxyl group (-COOH), carboxylate ($-COO^-$), an amide ($-CONH_2$) or an ester (-COOR).

As in the receptor protein of the present invention described above, the partial peptide of the present invention further includes those in which the amino group of the amino acid residue of the N-terminal methionine residue is protected by a protecting group, those in which the N-terminal residue is cleaved in vivo and the produced Gln is pyroglutaminated, those in which substituents on the side chains of amino acids in the molecule are protected by appropriate protecting groups, conjugated peptides such as so-called glycopeptides, to which sugar chains are bound, and the like.

For salts of the receptor protein of the present invention or its partial peptide, preferred are physiologically acceptable salts with acids or bases, especially physiologically acceptable acid addition salts. Examples of the salts include salts with, for example, inorganic acids (e.g., hydrochloric acid, phosphoric acid, hydrobromic acid, sulfuric acid); salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The partial peptide or its salts of the present invention can be manufactured by publicly known methods for peptide synthesis, or by cleaving the GPCR of the present invention with an appropriate peptidase. For the methods for peptide synthesis, for example, either solid phase synthesis or liquid phase synthesis may be used. That is, the partial peptide or amino acids that can construct the GPCR of the present invention are condensed with the remaining part. Where the product contains protecting groups, these protecting groups are removed to give the desired peptide. Publicly known methods for condensation and elimination of the protecting groups are described in a) - e) below.

a) M. Bodanszky & M.A. Ondetti: Peptide Synthesis, Interscience Publishers, New York (1966)
b) Schroeder & Luebke: The Peptide, Academic Press, New York (1965)

c) Nobuo Izumiya, et al.: Peptide Gosei-no-Kiso to Jikken (Basics and experiments of peptide synthesis), published by Maruzen Co. (1975)

d) Haruaki Yajima & Shunpei Sakakibara: Seikagaku Jikken Koza (Biochemical Experiment) 1, Tanpakushitsu no Kagaku (Chemistry of Proteins) IV, 205 (1977)

e) Haruaki Yajima, ed.: Zoku Iyakuhin no Kaihatsu (A sequel to Development of Pharmaceuticals), Vol. 14, Peptide Synthesis, published by Hirokawa Shoten

After completion of the reaction, the product may be purified and isolated by a combination of conventional purification methods such as solvent extraction, distillation, column chromatography, liquid chromatography and recrystallization to give the partial peptide of the present invention. When the partial peptide obtained by the methods above is in a free form, the peptide can be converted into an appropriate salt by a publicly known method; conversely when the peptide is obtained in a salt form, it can be converted into a free form by a publicly known method.

(Polynucleotide)

The polynucleotide encoding the receptor protein of the present invention may be any polynucleotide so long as it contains the base sequence (DNA or RNA, preferably DNA) encoding the receptor protein of the present invention described above. Such a polynucleotide may also be any one of DNA encoding the receptor protein of the present invention, or RNA such as mRNA, etc., and may be double-stranded or single-stranded. Where the polynucleotide is double-stranded, it may be double-stranded DNA, double-stranded RNA or DNA:RNA hybrid. Where the polynucleotide is single-stranded, it may be a sense strand (i.e., a coding strand) or an antisense strand (i.e., a non-coding strand).

Using the polynucleotide encoding the receptor protein of the present invention, mRNA of the receptor protein of the present invention can be quantified by, for example, the publicly known method published in separate volume of Jikken Igaku 15 (7) "New PCR and its application" (1997), or by its modifications.

The DNA encoding the receptor protein of the present invention may be any of genomic DNA, genomic DNA library, cDNA derived from the cells and tissues described above, cDNA library derived from the cells and tissues described above and synthetic DNA. The vector to be used for the library may be any of bacteriophage, plasmid, cosmid and phagemid. The DNA may also be directly amplified by reverse transcriptase polymerase chain reaction (hereinafter abbreviated as RT-PCR) using the total RNA or mRNA fraction prepared from the cells and tissues described above.

Specifically, the DNA encoding the receptor protein of the present invention may be any DNA, so long as it has, for example, a DNA hybridizable to a DNA comprising the base sequence represented by SEQ ID NO: 2 under high stringent conditions and encoding a receptor protein which has the activities substantially equivalent to those of the receptor protein comprising the amino acid sequence represented by SEQ ID NO: 2 (e.g., cell regeneration, growth or differentiation activities, ligand-binding activities, signal transduction actions, etc.).

Examples of the DNA hybridizable to the DNA comprising the base sequence represented by SEQ ID NO: 2 under high stringent conditions include a DNA comprising a base sequence having at least about 70% homology, preferably at least about 80% homology, more preferably at least about 90% homology and most preferably at least about 95% homology, to the base sequence represented by SEQ ID NO: 2.

The hybridization can be carried out by publicly known methods or by modifications of these methods, for example, according to the method described in Molecular Cloning, 2nd (J. Sambrook et al., Cold Spring Harbor Lab. Press, 1989). A commercially available library may also be used according to the instructions of the attached manufacturer's protocol. Preferably, the hybridization can be carried out under high stringent conditions.

The "high stringent conditions" used herein are, for example, those in a sodium concentration at about 19 to 40 mM, preferably about 19 to 20 mM at a temperature of about 50 to 70°C, preferably about 60 to 65°C. In particular, hybridization conditions in a sodium concentration of about 19 mM at a temperature of about 65°C are most preferred.

The "polynucleotide comprising a part of the base sequence of the DNA encoding the receptor protein of the present invention or a part of the base sequence complementary to the DNA" is used to mean that the polynucleotide embraces not only the DNA encoding the partial peptide of the present invention described below but also RNA.

According to the present invention, antisense polynucleotides (nucleic acids) that can inhibit the replication or expression of the receptor protein genes can be designed and synthesized based on the base sequence information of the cloned or determined DNA encoding the receptor protein. Such a polynucleotide (nucleic acid) is capable of hybridizing to RNA of the receptor protein gene to inhibit the synthesis or function of said RNA or capable of modulating or controlling the expression of the receptor protein gene via interaction with the receptor protein-associated RNA. Polynucleotides complementary to the selected sequences of the receptor protein-associated RNA and polynucleotides specifically hybridizable to the receptor protein-associated RNA are useful in modulating or controlling the expression of the receptor protein gene in vivo and in vitro, and useful for the treatment or diagnosis of diseases. The term "corresponding" is used to mean homologous to or complementary to a particular sequence of the nucleotide, base sequence or nucleic acid including the gene. The term "corresponding" between nucleotides, base sequences or nucleic acids and peptides (proteins) usually refer to amino acids of a peptide (protein) under the order derived from the sequence of nucleotides (nucleic acids) or their complements. In the receptor protein gene, the 5' end hairpin loop, 5' end 6-base-pair repeats, 5' end untranslated region, polypeptide translation initiation codon, protein coding region, translation termination codon, 3' end untranslated region, 3' end palindrome region, and 3' end hairpin loop, may be selected as preferred target regions, though any other region may be selected as a target in the receptor protein gene.

For cloning of the DNA that completely encodes the receptor protein of the present invention or its partial peptide (hereinafter sometimes collectively referred to as the receptor protein of the present invention), the DNA may be amplified by PCR using synthetic DNA primers containing a part of the base sequence of the receptor protein of the present invention, or the DNA inserted into an appropriate vector can be selected by hybridization with a labeled DNA fragment or synthetic DNA that encodes a part or entire region of the receptor protein of the present invention. The hybridization can be carried out, for example, according to the method described in Molecular Cloning, 2nd, J. Sambrook et al., Cold Spring Harbor Lab. Press, 1989. The hybridization may also be performed using commercially available library in accordance with the protocol described in the attached instructions.

(Antisense Polynucleotide)

The relationship between the targeted nucleic acids and the hybridizable polynucleotides complementary to at least a part of the target or the relation to the polynucleotides hybridizable to the target can be denoted to be "antisense" to the target. Examples of the antisense polynucleotides include polynucleotides containing 2-deoxy-D-ribose, polynucleotides containing D-ribose, any other type of polynucleotides which are N-glycosides of a purine or pyrimidine base, or other polymers containing non-nucleotide backbones (e.g., protein nucleic acids and synthetic sequence-specific nucleic acid polymers commercially available) or other polymers containing nonstandard linkages (provided that the polymers contain nucleotides having such a configuration that allows base pairing or base stacking, as is found in DNA or RNA), etc. They may be double-stranded DNA, single-stranded DNA, double-stranded RNA, single-stranded RNA or a DNA:RNA hybrid, and may further include unmodified polynucleotides (or unmodified oligonucleotides), those with publicly known types of modifications, for example, those with labels known in the art, those with caps, methylated polynucleotides, those with substitution of one or more naturally occurring nucleotides by their analogue, those with intramolecular modifications of nucleotides such as those with uncharged linkages (e.g., methyl phosphonates, phosphotriesters, phosphoramidates, carbamates, etc.) and those with charged linkages or sulfur-containing linkages (e.g., phosphorothioates, phosphorodithioates, etc.), those having side chain groups such as proteins (nucleases, nuclease inhibitors, toxins, antibodies, signal peptides, poly-L-lysine, etc.), saccharides (e.g., monosaccharides, etc.), those with intercalators (e.g., acridine, psoralen, etc.), those containing chelators (e.g., metals, radioactive metals, boron, oxidative metals, etc.), those containing alkylating agents, those with modified linkages (e.g., anomeric nucleic acids, etc.), and the like. Herein the terms "nucleoside", "nucleotide" and "nucleic acid" are used to refer to moieties that contain not only the purine and pyrimidine bases, but also other heterocyclic bases, which have been modified. Such modifications may include methylated purines and pyrimidines, acylated purines and pyrimidines and other heterocyclic rings. Modified nucleotides and modified nucleotides also include modifications on the sugar moiety, wherein, for example, one or more hydroxyl groups may optionally be substituted with a halogen atom(s), an aliphatic group(s), etc., or may be converted into the corresponding functional groups such as ethers, amines, or the like.

The antisense polynucleotide (nucleic acid) of the present invention is RNA, DNA or a modified nucleic acid (RNA, DNA). Specific examples of the modified nucleic acid are, but not limited to, sulfur and thiophosphate derivatives of nucleic acids and those resistant to degradation of polynucleoside amides or oligonucleoside amides. The antisense nucleic acids of the present invention can be modified preferably based on the following design, that is, by increasing the intracellular stability of the antisense nucleic acid, increasing the cellular permeability of the antisense nucleic acid, increasing the affinity of the nucleic acid to the targeted sense strand to a higher level, or minimizing the toxicity, if any, of the antisense nucleic acid.

Many of such modifications are known in the art, as disclosed in J. Kawakami, et al., Pharm. Tech. Japan, Vol. 8, pp. 247, 1992; Vol. 8, pp. 395, 1992; S. T. Crooke, et al. ed., Antisense Research and Applications, CRC Press, 1993; etc.

The antisense nucleic acid of the present invention may contain changed or modified sugars, bases or linkages. The antisense nucleic acid may also be provided in a specialized form such as liposomes, microspheres, or may be applied to gene therapy, or may be provided in combination with attached moieties. Such attached moieties include polycations such as polylysine that act as charge neutralizers of the phosphate backbone, or hydrophobic moieties such as lipids (e.g., phospholipids, cholesterols, etc.) that enhance the interaction with cell membranes or increase uptake of the nucleic acid. Preferred examples of the lipids to be attached are cholesterols or derivatives thereof (e.g., cholesteryl chloroformate, cholic acid, etc.). These moieties may be attached to the nucleic acid at the 3' or 5' ends thereof and may also be attached thereto through a base, sugar, or intramolecular nucleoside linkage. Other moieties may be capping groups specifically placed at the 3' or 5' ends of the nucleic acid to prevent degradation by nucleases such as exonuclease, RNase, etc. Such capping groups include, but are not limited to, hydroxyl protecting groups known in the art, including glycols such as polyethylene glycol, tetraethylene glycol and the like.

The inhibitory action of the antisense nucleic acid can be examined using the transformant of the present invention, the gene expression system of the present invention in vivo and in vitro, or the translation system of the receptor protein in vivo and in vitro. The nucleic acid can be applied to cells by a variety of publicly known methods.

Since the antisense polynucleotide of the present invention can suppress the function of the protein of the present invention or the polynucleotide (such as DNA) of the present invention in vivo, it can be used for example as a preventive and/or therapeutic agents for disorders associated with dysfunction of the receptor protein of the present invention. Moreover, since the antisense polynucleotide of the present invention can also be used as a diagnostic oligonucleotide probe for investigating the presence and expression of the DNA of the present invention in tissue and cells, it can be used for diagnosing disorders associated with dysfunction of the receptor protein of the present invention.

(DNA Encoding Partial Peptide)

The DNA encoding the partial peptide of the present invention may be any DNA so long as it contains the base sequence encoding the partial peptide of the present invention described above. The DNA may also be any of genomic DNA, genomic DNA library, cDNA derived from the cells and tissues described above, cDNA library derived from the cells and tissues described above and synthetic DNA. The vector to be used for the library may be any of bacteriophage, plasmid, cosmid and phagemid. The DNA may also be directly amplified by reverse transcriptase polymerase chain reaction (hereinafter abbreviated as RT-PCR) using mRNA fraction prepared from the cells and tissues described above.

Specifically, the DNA encoding the partial peptide of the present invention may be any one of, for example,

(1) a DNA having a partial base sequence of the DNA comprising the base sequence represented by SEQ ID NO: 2, or
(2) a DNA having a DNA hybridizable to the DNA comprising the base sequence represented by SEQ ID NO: 2 under highly stringent conditions and having a partial base sequence of the DNA encoding the protein which has the activities (e.g., cell regeneration, differentiation or growth activities, ligand-biding activities, signal transduction actions, etc.) substantially equivalent to those of the protein peptide comprising the amino acid sequences represented by SEQ ID NO: 1; etc.

Examples of the DNA that is hybridizable to a DNA comprising the base sequence represented by SEQ ID NO: 2 include a DNA comprising a base sequence having at least about 70% homology, preferably at least about 80% homology, more preferably at least about 90% homology, and even more preferably at least about 95% homology, to the base sequence represented by SEQ ID NO: 2.

(Antibody)

Antibodies to the receptor protein of the present invention or its partial peptide, or salts thereof may be either polyclonal antibodies or monoclonal antibodies as long as they are capable of recognizing the receptor protein of the present invention or its partial peptide or salts thereof, or cells or tissues containing the receptor protein of the present invention.

The antibodies to the receptor protein of the present invention or its partial peptide, or salts thereof (hereinafter sometimes merely referred to as the receptor protein, etc. of the present invention) can be manufactured by publicly known methods for manufacturing antibodies or antisera, using as antigens the receptor protein, etc. of the present invention.

[Preparation of Monoclonal Antibody]

(a) Preparation of monoclonal monoclonal antibody-producing cells

The receptor protein, etc. of the present invention is administered to mammals either solely or together with carriers or diluents to the site where the production of antibody is possible by the administration. In order to potentiate the antibody productivity upon the administration, complete Freund's adjuvants or incomplete Freund's adjuvants may be administered. The administration is usually carried out once in every two to six weeks and 2 to 10 times in total. Examples of the applicable mammals are monkeys, rabbits, dogs, guinea pigs, mice, rats, sheep and goats, with mice and rats being preferred.

In producing the monoclonal antibody-producing cells, warm-blooded animals, e.g., mice, immunized with an antigen wherein the antibody titer is noted is selected, then the spleen or lymph node is collected after 2 to 5 days from the final immunization and antibody-producing cells contained therein are fused with myeloma cells to give monoclonal antibody-producing hybridomas. Measurement of the antibody titer in antisera may be made, for example, by reacting a labeled form of the receptor protein, etc., which will be described later, with the antiserum followed by assaying the binding activity of the labeling agent bound to the antibody. The fusion may be operated, for example, by the known Koehler and Milstein method [Nature, 256, 495 (1975)]. Examples of the fusion accelerator are polyethylene glycol (PEG), Sendai virus, etc., of which PEG is preferably employed.

Examples of the myeloma cells are NS-1, P3U1, SP2/0, etc. In particular, P3U1 is preferably employed. A preferred ratio of the count of the antibody-producing cells used (spleen cells) to the count of myeloma cells is within a range of approximately 1:1 to 20:1. When PEG (preferably, PEG 1000 to PEG 6000) is added in a concentration of approximately 10 to 80% followed by incubating at about 20 to about 40°C, preferably at about 30 to about 37°C for about 1 to about 10 minutes, an efficient cell fusion can be carried out.

Various methods can be used for screening of a monoclonal antibody-producing hybridoma. Examples of such methods include a method which comprises adding the supernatant of hybridoma to a solid phase (e.g., microplate) adsorbed with an antigen of the receptor protein, etc. directly or together with a carrier, adding an anti-immunoglobulin antibody (when mouse cells are used for the cell fusion, anti-mouse immunoglobulin antibody is used) labeled with a radioactive substance or an enzyme, or Protein A and detecting the monoclonal antibody bound to the solid phase, and a method which comprises adding the supernatant of hybridoma to a solid phase adsorbed with an anti-immunoglobulin antibody or Protein A, adding the receptor protein, etc. labeled with a radioactive substance or an enzyme and detecting the monoclonal antibody bound to the solid phase.

The monoclonal antibody can be selected by publicly known methods or by modifications of these methods. In general, the selection can be effected in a medium for animal cells supplemented with HAT (hypoxanthine, aminopterin and thymidine). Any selection and growth medium can be employed as far as the hybridoma can grow therein. For example, RPMI 1640 medium containing 1% to 20%, preferably 10% to 20% fetal bovine serum, GIT medium (Wako Pure Chemical Industries, Ltd.) containing 1% to 10% fetal bovine serum, a serum free medium for cultivation of a hybridoma (SFM-101, Nissui Seiyaku Co., Ltd.) and the like can be used for the selection and growth medium. The cultivation is carried out generally at 20°C to 40°C, preferably at about 37°C, for 5 days to 3 weeks, preferably 1 to 2 weeks. The cultivation can be conducted normally in 5% $CO_2$. The antibody titer of the culture supernatant of hybridomas can be determined as in the assay for the antibody titer in antisera described above.

(b) Purification of monoclonal antibody

Separation and purification of the monoclonal antibody can be carried out by methods applied to conventional separation and purification of immunoglobulins, as in conventional methods for separation and purification of polyclonal antibodies [e.g., salting-out, alcohol precipitation, isoelectric point precipitation, electrophoresis, adsorption and desorption with ion exchangers (e.g., DEAE), ultracentrifugation, gel filtration, or a specific purification method which comprises collecting only an antibody with an activated adsorbent such as an antigen-binding solid phase, Protein A, Protein G, etc. and dissociating the binding to obtain the antibody].

[Production of Polyclonal Antibody]

The polyclonal antibody of the present invention can be manufactured by publicly known methods or modifications thereof For example, a complex of immunogen (antigen such as the protein, etc. of the present invention) and a carrier protein is prepared, and a mammal is immunized with the complex in a manner similar to the method described above for the manufacture of monoclonal antibodies. The product containing the antibody to the receptor protein, etc. of the present invention is collected from the immunized animal followed by separation and purification of the antibody.

In the complex of an immunogen and a carrier protein used to immunize a mammal, the type of carrier protein and the mixing ratio of a carrier to hapten may be any type and in any ratio, as long as the antibody is efficiently produced to the hapten immunized by crosslinking to the carrier. For example, bovine serum albumin, bovine thyroglobulins, keyhole limpet hemocyanin, etc. is coupled to hapten in a carrier-to-hapten weight ratio of approximately 0.1 to 20, preferably about 1 to about 5.

A variety of condensing agents can be used for the coupling of a carrier to hapten. Glutaraldehyde, carbodiimide, maleimide activated ester, activated ester reagents containing thiol group or dithiopyridyl group, etc. are used for the coupling.

The condensation product is administered to warm-blooded animals either solely or together with carriers or diluents to the site in which the antibody can be produced by the administration. In order to potentiate the antibody productivity upon the administration, complete Freund's adjuvant or incomplete Freund's adjuvant may be administered. The administration is usually made once approximately in every 2 to 6 weeks and about 3 to about 10 times in total.

The polyclonal antibody can be collected from the blood, ascites, etc., preferably from the blood of mammals immunized by the method described above.

The polyclonal antibody titer in antiserum can be assayed by the same procedure as that for the determination of serum antibody titer described above. The separation and purification of the polyclonal antibody can be carried out, following the method for the separation and purification of immunoglobulins performed as applied to the separation and purification of monoclonal antibodies described hereinabove.

[Use of Receptor Protein, DNA, etc.]

The receptor protein of the present invention, the polynucleotides encoding the same (hereinafter sometimes briefly referred to as the polynucleotide of the present invention), the compounds acting on the receptor protein of the present invention (ligands, agonists, antagonists, inhibitors, activators, antibodies, etc.), the antibodies to the receptor protein of the present invention (hereinafter sometimes briefly referred to as the antibodies of the present invention), the antisense polynucleotides to the DNA of the present invention (hereinafter sometimes merely referred to as the antisense polynucleotide of the present invention) and the like have the following uses. Cholecystokinin is also known as the ligand to the receptor protein of the present invention.

(1) Agent for Prevention and/or Treatment of Disorders Associated with Cell Regeneration, Growth or Differentiation

Since the receptor protein of the present invention is associated with cell regeneration, growth or differentiation, a) the receptor protein of the present invention, b) the polynucleotide encoding the receptor protein of the present invention (e.g., DNA), and c) the compound acting on the receptor protein of the present invention (ligand, agonist, antagonist, inhibitor, activator, antibody, etc.) are useful as the regulators for cell regeneration, growth or differentiation. Therefore, the regulators for cell regeneration, growth or differentiation can be used as agents for the prevention and/or treatment of disorders associated with cell regeneration, growth or differentiation. Such disorders include, for example, disorders or injuries such as obesity, mellitus diabetes mellitus, immune system disorders (e.g., autoimmune disease, atopic dermatitis, allergic diseases, immunodeficiency, asthma, rheumatoid arthritis, psoriasis, arterial sclerosis, diabetes mellitus, Alzheimer's disease, etc.), liver disorders (e.g., hepatic cirrhosis, hepatitis, hepatic insufficiency, etc.), alimentary disorders (e.g., ulcer, enteritis, indigestion, irritable bowel syndrome, ulcerative colitis, diarrhea, ileus, etc.), tissue damages in cosmetic surgery, and the like. Furthermore, by inhibiting or activating the protein comprising the same or substantially the same amino acid sequence as the amino acid sequence represented by SEQ ID NO: 1, the regeneration, growth or differentiation of a cell containing the aforesaid protein can be regulated.

For example, where the physiological functions (e.g., cell regeneration, growth or differentiation activities) of a ligand cannot be relied upon because the receptor protein of the present invention is reduced in the body of a patient (deficiency of the receptor protein of the present invention), the functions of the ligand can be sufficiently exerted by a) administering the receptor protein of the present invention to the patient to replenish the level of said receptor protein, or b) increasing the level of said receptor protein in the body of the patient either (i) by administering DNA encoding the receptor protein of the present invention to the patient to induce expression, or (ii) by inserting DNA encoding the receptor protein of the present invention into target cells and transplanting the cells into the patient to induce expression.

Since cholecystokinin is a ligand for the receptor protein of the present invention, cholecystokinin and its analogs can be used as the regulator for cell regeneration, growth or differentiation. Such a regulator for cell regeneration, growth or differentiation is capable of regulating the differentiation of stem cells, especially mesenchymal stem cells, and is useful. In addition, it is confirmed that such a regulator for cell regeneration, growth or differentiation can regulate the differentiation into adipocytes (see EXAMPLE 2 later described) and is useful also as a regulator for differentiation into adipocytes.

When the receptor protein of the present invention, the polynucleotide encoding the same, the compound acting on the receptor protein of the present invention, and the like, are used as the medicaments described above, they can be prepared into pharmaceutical preparations in a conventional manner and the pharmaceutical preparations can be administered in a manner as described above.

(2) Diagnostic Agent and Method for Diagnosis Using Polynucleotide or Antisense Polynucleotide

By using as probes the polynucleotide (e.g., DNA) and antisense polynucleotide (e.g., antisense DNA) which can be identified by the method of the present invention, an abnormality (gene abnormality) of the DNA or mRNA encoding the receptor protein of the present invention or its partial peptide can be detected in human or mammal (e.g., rat, mouse, rabbit, sheep, swine, bovine, cat, dog, monkey, etc.). Therefore, the polynucleotide and antisense polynucleotide are useful as gene diagnostic agents for detecting damages to the DNA or mRNA, its mutation, or decreased expression, increased expression, overexpression, etc. of the DNA or mRNA.

The gene diagnosis described above using the polynucleotide or antisense polynucleotide of the present invention can be performed by, for example, the publicly known northern hybridization assay or the PCR-SSCP assay (Genomics, 5, 874-879 (1989); Proceedings of the National Academy of Sciences of the United States of America, 86, 2766-2770 (1989)), etc.

When decreased expression or overexpression of the receptor protein of the present invention is detected by, e.g., northern hybridization, it can be diagnosed that it is highly likely to suffer from, for example, disorders caused by the dysfunction or overexpression of the receptor protein of the present invention, for instance, diseases such as obesity, diabetes mellitus, immune system disorders (e.g., autoimmune disease, atopic dermatitis, allergic diseases, immunodeficiency, asthma, rheumatoid arthritis, psoriasis, arterial sclerosis, diabetes mellitus, Alzheimer's disease, etc.), liver/gallbladder disorders (e.g., hepatic cirrhosis, hepatitis, hepatic insufficiency, cholestasis, calculus, etc.), alimentary disorders (e.g., ulcer, enteritis, indigestion, irritable bowel syndrome, ulcerative colitis, diarrhea, ileus, etc.), heat burn, fracture, alopecia, etc.; or it is highly likely to suffer from these disease in the future.

(3) Medicament Comprising Antisense Polynucleotide

The antisense polynucleotide which is identifiable by the method of the present invention can be used as an agent for the prevention and/or treatment of disorders caused by overexpression, etc. of the receptor protein of the present invention (e.g., neurological disorders, inflammatory disorders, circulatory disorders, cancer, obesity, diabetes mellitus, immune disorders, liver/gallbladder disorders, alimentary disorders, heat burn, fracture, osteoarthritis, periodontal disease or alopecia).

For example, in the case where the antisense polynucleotide is used, the antisense polynucleotide itself is administered; alternatively, the antisense polynucleotide is inserted into an appropriate vector such as retrovirus vector, adenovirus vector, adenovirus-associated virus vector, etc. and then administered in a conventional manner. The antisense polynucleotide can be prepared in a pharmaceutical preparation as it is or together with a physiologically acceptable carrier such as a coadjuvant for intake enhancement and subsequently can be administered using a gene gun or a catheter such as a hydrogel catheter.

In addition, the antisense polynucleotide can be used as a diagnostic oligonucleotide probe to examine the presence of the DNA of the present invention in tissues or cells and an aspect of its expression.

4) Method of Screening Compound that Alters Expression Level of Gene Products of Gene Identified by the Aforesaid Screening Method, Receptor Protein of Present Invention or Its Partial Peptide

By using as a probe a polynucleotide (e.g., the DNA) having the base sequence of the gene identified by the screening method described above, the polynucleotide can be used for screening the compound that alters the expression level of the gene products of the gene identified by the screening method described above, the receptor protein of the present invention or its partial peptide. Namely, the compound which regulates cell regeneration, growth or differentiation can be screened.

That is, the present invention provides a method of screening the compound that alters the expression level of the gene identified by the screening method described above, the receptor protein of the present invention or its partial peptide, which comprises measuring the level of mRNA for the receptor protein of the present invention or its partial peptide contained, for example, in (i) (1) blood, (2) particular organs, (3) tissues or cells isolated from the organs of non-human mammals or in (ii) transformants, etc.

Specifically, the level of mRNA for the gene products of the gene identified by the screening method described above, the receptor protein of the present invention or its partial peptide can be measured as follows.

(i) Normal or disease models of non-human mammals (e.g., mice, rats, rabbits, sheep, swine, bovine, cats, dogs, monkeys, etc., more specifically, tumor-bearing mice, etc.) receive administration of a drug (e.g., anticancer drug, etc.) or physical stress (e.g., soaking stress, electric shock, light and darkness, low I temperature, etc.), and blood, particular organs (e.g., brain, lung, colon, prostate, etc.), or tissues or cells isolated from the organs are obtained after a specified period of time.

The mRNA for the receptor protein of the present invention or its partial peptide contained in the thus obtained cells is extracted from the cells, e.g., in a conventional manner and quantified by means of, e.g., TaqMan PCR, or may also be analyzed by northern blot technique by publicly known methods.

(ii) Transformants which express the gene identified by the screening method described above or its gene products, or the receptor protein of the present invention or its partial peptide are prepared according to the methods described above, and the mRNA of the receptor protein of the present invention or its partial peptide, which is contained in said transformants, can be quantified and analyzed as described above.

The compound that alters the expression level of the gene identified by the screening method described above or its gene products, or the receptor protein of the present invention or its partial peptide (the compound that acts on the receptor protein of the present invention) can be screened by the following procedures.

(i) To normal or disease models of non-human mammals, a test compound is administered at a specified period of time before (30 minutes to 24 hours before, preferably 30 minutes to 12 hours before, more preferably 1 hour to 6 hours before), at a specified time after (30 minutes to 3 days after, preferably 1 hour to 2 days after, more preferably 1 hour to 24 hours after), or simultaneously with a drug or physical stress. At a specified time (30 minute to 3 days, preferably 1 hour to 2 days, more preferably 1 hour to 24 hours) after administration of the test compound, the amount of mRNA for the receptor protein of the present invention or its partial peptide contained in cells are quantified and analyzed.
(ii) When transformants are cultured in a conventional manner, a test compound is mixed in a culture medium. After incubation for a given time period (after 1 day to 7 days, preferably after 1 day to 3 days, more preferably after 2 to 3 days), the level of mRNA for the receptor protein of the present invention or its partial peptide contained in the transformants can be quantified and analyzed.

The compounds or their salts, which are obtained by the screening methods of the present invention, are compounds that have an activity to alter the expression level of the gene identified by the screening method described above or its gene products, or the receptor protein of the present invention or its partial peptide. Specifically, (a) compounds that potentiate the cell regeneration, growth or differentiation activity by increasing the expression level of the gene identified by the screening method described above or its gene products, or the receptor protein of the present invention or its partial peptide; and (b) compounds that decrease the cell regeneration, growth or differentiation activity by reducing the expression level of the gene identified by the screening method described above or its gene products, or the receptor protein of the present invention or its partial peptide.
The test compounds include peptides, proteins, non-peptide compounds (lipids, amines, amino acids, sugars, nucleic acids, ions, etc.), synthetic compounds, and fermentation products. These compounds may be novel compounds or may be publicly known compounds.
The compounds capable of regulating the cell regeneration, growth or differentiation activity obtained as described above is useful as the regulator for cell regeneration, growth or differentiation.

(5) Preventive and/or Therapeutic Agent for Various Disorders Comprising Compound which Alters Expression Level of Receptor Protein of Invention or Its Partial Peptide

As described above, the receptor protein of the present invention is considered to serve some important role in vivo, for example, in the central system function or the like. Consequently, the compound that alters the expression level of the receptor protein of the present invention or its partial peptide can be used as an agent for the prevention and/or treatment of disorders associated with dysfunction of the receptor protein of the present invention.
When said compound is used as an agent for the prevention and/or treatment of disorders associated with dysfunction of the receptor protein of the present invention, it can be formulated in a conventional manner as described above.

(6) Screening Method and Screening Kit for Compound (Agonist, Antagonist, Inhibitor, Activator, etc.) Capable of Regulating Cell Regeneration, Growth or Differentiation Using Receptor Protein of Invention

The receptor protein of the present invention is associated with cell regeneration, growth or differentiation, and the compound that alters the binding property of this protein to its ligand is assumed to be capable of regulating cell regeneration, growth or differentiation. Accordingly, compounds for regulating cell regeneration, growth or differentiation can be subjected to binding tests for primary screening. Whether the compound selected by this screening has the cell regeneration, growth or differentiation activity or not can be readily confirmed by actually contacting the compound with a cell having the regeneration, differentiation or growth capability.
By using the receptor protein, etc. of the present invention or by constructing a system for expressing a recombinant receptor protein, etc. and using the receptor-binding assay system employing said expression system, for compounds (e.g., peptides, proteins, non-peptide compounds, synthetic compounds, fermentation products, etc.) or salts thereof that alter the binding properties of the receptor protein, etc. of the present invention to the ligand can be efficiently screened. Kinds of the ligands as used herein are not particularly limited and are, for example, proteins, peptides, lipids, amines, amino acids, sugars, nucleic acids and ions, including naturally occurring agonists and antagonists, recombinant agonists and antagonists, synthetic agonists and antagonists, their derivatives and metabolites, biosynthesis inhibitors of the ligand, inhibitors on the signal transduction system, neutralizing antibodies and binding proteins.
As described above, one example of the ligands for the receptor protein of the present invention is cholecystokinin.

The compounds that alter the binding properties of the ligand to the receptor protein of the present invention include (a) compounds having an activity to promote or an activity to suppress the receptor-mediated cell stimulating activity (so-called agonists to the receptor protein of the present invention), (b) compounds having no such cell stimulating activity (so-called antagonists to the receptor protein of the present invention), (c) compounds which potentiate the binding affinity of the ligand to the G protein-coupled receptor protein of the present invention, (d) compounds which reduce the binding affinity of the ligand to the receptor protein of the present invention (the compounds (a) described above is preferably screened by the aforesaid ligand determination method), and the like.

The cell stimulating activities include, for,example, (1) arachidonic acid release, (2) acetylcholine release, (3) intracellular $Ca^{2+}$ release, (4) intracellular cAMP production, (5) intracellular cGMP production, (6) inositol phosphate production, (7) cell membrane potential changes, (8) phosphorylation of intracellular proteins (such as MAP kinase), (9) activation of c-fos, (10) reduction of pH, (11) activation of low molecular weight G proteins such as Rho, Rac, Ras, etc., and (12) activation of reporter genes (such as luciferase) ligated downstream the transcription factor CRE (cAMP responsive element), AP1, NFAT, SRE (serum responsive element) or the like, with particularly preferred being the intracellular cAMP production-promoting activity, etc.

That is, the present invention provides a method of screening a compound or its salt that alters the binding properties of the receptor protein of the present invention, its partial peptide, or salts thereof, to the ligand, which comprises comparing the following two cases: (i) the case wherein the receptor protein of the present invention, its partial peptide, or salts thereof are brought in contact with the ligand; and (ii) the case wherein the receptor protein of the present invention, its partial peptide or the salts thereof are brought in contact with the ligand and a test compound.

The screening methods of the present invention is characterized by determining and comparing, for example, the binding amounts of the ligand to the receptor protein, etc., the cell stimulating activities, etc. between the cases (i) and (ii).

More specifically, the present invention provides the following screening methods.

(i) A method of screening a compound or its salt that alters the binding properties of a ligand to the receptor protein, etc. of the present invention, which comprises assaying the binding amount of a labeled ligand to the receptor protein, etc. in the case where a labeled form of the ligand is brought in contact with the receptor protein, etc. of the present invention and in the case where a labeled form of the ligand and a test compound are brought in contact with the receptor protein, etc. of the present invention, and comparing the binding amounts.

(ii) A method of screening a compound or its salt that alters the binding properties of a ligand to the receptor protein, etc. of the present invention, which comprises assaying the binding amount of a labeled ligand to a cell containing the receptor protein, etc. of the present invention or a membrane fraction of the cell, in the case where a labeled form of the ligand is brought in contact with the cell or the membrane fraction and in the case where a labeled form of the ligand and a test compound are brought in contact with the cell containing the receptor protein, etc. of the present invention or its membrane fraction, and comparing the binding amounts.

(iii) A method of screening a compound or its salt that alters the binding properties of a ligand to the receptor protein, etc., which comprises assaying the binding amount of a labeled ligand to the receptor protein, etc. of the present invention, in the case where a labeled form of the ligand is brought in contact with the receptor protein, etc. of the present invention expressed on a cell membrane by culturing a transformant containing the DNA of the present invention and in the case where a labeled form of the ligand and a test compound are brought in contact with the receptor protein, etc. of the present invention expressed on the cell membrane by culturing a transformant containing the DNA of the present invention, and comparing the binding amounts.

(iv) A method of screening a compound or its salt that alters the binding properties of a ligand to the receptor protein, etc. of the present invention, which comprises assaying the receptor-mediated cell stimulating activities, in the case where a compound (e.g., the ligand, etc.) that activates the receptor protein, etc. of the present invention is brought in contact with a cell containing the receptor protein, etc. of the present invention and in the case where said compound that activates the receptor protein, etc. of the present invention and a test compound are brought in contact with the cell containing the receptor protein, etc. of the present invention, and comparing the cell stimulating activities.

(v) A method of screening a compound or its salt that alters the binding properties of a ligand to the receptor protein, etc. of the present invention, which comprises assaying the receptor-mediated cell stimulating activities in the case where a compound (e.g., the ligand, etc.) that activates the receptor protein, etc. of the present invention is brought in contact with the receptor protein, etc. of the present invention expressed on a cell membrane by culturing a transformant containing the DNA of the present invention and in the case where said compound that activates the receptor protein, etc. of the present invention and a test compound are brought in contact with the receptor protein, etc. of the present invention expressed on a cell membrane by culturing a transformant containing the DNA of the present invention, and comparing the cell stimulating activities.

Hereinafter the screening methods of the present invention will be described more specifically.

First, the receptor protein, etc. of the present invention, which is used for the screening methods of the present invention, may be any one so long as it contains the receptor protein, etc. of the present invention described above, though membrane fractions from mammalian organs containing the receptor protein, etc. of the present invention are preferably employed. Since it is very difficult to obtain human-derived organs especially, the human-derived receptor protein, etc. expressed abundantly by recombinants are suitable for use in the screening.

To produce the receptor protein of the present invention, the methods described above can be used, and the DNA of the present invention is preferably expressed on mammalian cells or insect cells. As the DNA fragment encoding the objective protein region, a complementary DNA may be used but is not limited thereto. For example, gene fragments or a synthetic DNA may also be used. In order to introduce the DNA fragment encoding the receptor protein of the present invention into host animal cells and express the same efficiently, the DNA fragment is preferably incorporated into a polyhedron promoter of nuclear polyhedrosis virus (NPV) belonging to the Baculovirus whose host is insects, an SV40-derived promoter, a promoter of retrovirus, a metallothionein promoter, a human heat shock promoter, a cytomegalovirus promoter, SR$\alpha$ promoter, etc. at the downstream thereof. The quantity and quality of the thus expressed receptors can be examined by a publicly known method, for example, by the method described in the literature [Nambi, P. et al., J. Biol. Chem., 267, 19555-19559, 1992].

Accordingly, in the screening methods of the present invention, those having the receptor protein, etc. of the present invention may be receptor proteins, etc. purified by publicly known methods, or cells bearing the said receptor protein, etc. or membrane fractions of the cells bearing said receptor protein, etc. may be used as well.

When the cell containing the receptor protein, etc. of the present invention is used in the screening methods of the present invention, the cell may be fixed with glutaraldehyde, formalin, etc. The fixation may be carried out by a publicly known method.

The cell containing the receptor protein, etc. of the present invention refers to a host cell in which the receptor protein, etc. is expressed. Preferred examples of such a host cell include Escherichia coli, Bacillus subtilis, yeast, insect cells, animal cells, etc.

The membrane fraction of the cell means a fraction abundant in cell membrane obtained by cell disruption and subsequent fractionation by publicly known methods. Cell disruption methods include cell squashing using a Potter-Elvehjem homogenizer, disruption using a Waring blender or Polytron (manufactured by Kinematica Inc.), disruption by ultrasonication, disruption by cell spraying through thin nozzles under an increased pressure using a French press, or the like. Cell membrane fractionation is effected mainly by fractionation using a centrifugal force, such as centrifugation for fractionation and density gradient centrifugation. For example, cell disruption fluid is centrifuged at a low speed (500 rpm to 3,000 rpm) for a short period of time (normally about 1 to about 10 minutes), the resulting supernatant is then centrifuged at a higher speed (15,000 rpm to 30,000 rpm) normally for 30 minutes to 2 hours. The precipitate thus obtained is used as the membrane fraction. The membrane fraction is rich in the receptor protein, etc. expressed and membrane components such as cell-derived phospholipids, membrane proteins, etc.

The amount of the receptor protein in a cell containing said receptor protein, etc. or the cell membrane fraction is preferably $10^3$ to $10^8$ molecules per cell, more preferably $10^5$ to $10^7$ molecules per cell. As the level of expression increases, the ligand binding activity per unit of membrane fraction (specific activity) increases so that not only the highly sensitive screening system can be constructed but also large quantities of samples can be assayed with the same lot.

To perform (1) through (3) above for screening the compound that alters the binding properties of the ligand to the receptor protein, etc. of the present invention, an appropriate fraction of the receptor protein and a labeled form of the ligand are required.

For the receptor protein fraction, a naturally occurring receptor protein fraction or a recombinant receptor protein fraction having activities equivalent thereto. Herein, the equivalent activities refer to ligand binding activities, signal transduction activities, etc., which are equivalent.

For the labeled ligand, a labeled ligand and a labeled ligand analogue compound are used. For example, ligands labeled with [$^3$H], [$^{125}$I], [$^{14}$C], [$^{35}$S], etc. are used.

More specifically, for screening the compound that alters the binding properties of the ligand to the receptor protein, etc. of the present invention, a receptor protein preparation is prepared by suspending cells containing the receptor protein, etc. of the present invention or a membrane fraction of the cells in a buffer appropriate for use in the screening methods. Any buffer can be used so long as it does not interfere the ligand-receptor protein binding, including a phosphate buffer or a Tris-HCl buffer, having pH of 4 to 10 (preferably pH of 6 to 8), etc. For the purpose of minimizing non-specific binding, a surfactant such as CHAPS, Tween-80™ (Kao-Atlas Inc.), digitonin, deoxycholate, etc., may optionally be added to the buffer. Further for the purpose of suppressing the degradation of the receptor or ligand with a protease, a protease inhibitor such as PMSF, leupeptin, E-64 (manufactured by Peptide Institute, Inc.), pepstatin, etc. may also be added. A given amount (5,000 cpm to 500,000 cpm) of a labeled form of the ligand is added to 0.01 ml to 10 ml of the receptor protein solution, in which $10^{-4}$ M to $10^{-10}$ M of a test compound is co-present. To determine the amount of non-specific binding (NSB), a reaction tube charged with an unlabeled form of the ligand in a large excess is also provided. The reaction is carried out at approximately 0°C to 50°C, preferably 4°C to 37°C for 20 minutes to 24 hours, preferably 30 minutes to 3 hours. After completion of the reaction, the reaction mixture is filtrated through glass fiber filter paper, etc. and washed with an appropriate volume of the same buffer. The residual radioactivity on the glass fiber filter paper is then measured by means of a liquid scintillation counter or γ-counter. When nonspecific binding (NSB) is subtracted from the count ($B_0$) where any antagonizing substance is absent and the resulting count ($B_0$-NSB) is made 100%, the test compound showing the specific binding amount (B-NSB) of, e.g., 50% or less may be selected as a candidate compound.

To perform the methods of screening the compound that alters the binding properties of the ligand to the receptor protein, etc. of the present invention, for example, the aforesaid receptor protein-mediated cell stimulating activities can be assayed by a publicly known method, or using an assay kit commercially available.

Specifically, the cells containing the receptor protein, etc. of the present invention are first cultured in a multiwell plate, etc. Prior to screening, the medium is replaced with fresh medium or with an appropriate non-cytotoxic buffer, followed by incubation for a given period of time in the presence of a test compound, etc. Subsequently, the cells are extracted or the supernatant is recovered and the resulting product is quantified by appropriate procedures. Where it is difficult to detect the production of the cell-stimulating activity indicator (e.g., arachidonic acid, etc.) due to a degrading enzyme contained in the cells, an inhibitor against such as a degrading enzyme may be added prior to the assay. For detecting the activities such as the cAMP production suppression activity, the baseline production in the cells is increased by forskolin or the like and the suppressing effect on the increased baseline production can be detected.

For the screening through the assay for the cell stimulating activities, cells wherein an appropriate receptor protein has been expressed are required. Desired cells wherein the receptor protein, etc. of the present invention has been expressed are a cell line having the receptor protein, etc. of the present invention of naturally occurring type and a cell line wherein the aforesaid recombinant receptor protein, etc. described above has been expressed, etc.

Examples of the test compounds include peptides, proteins, antibodies, non-peptide compounds, synthetic compounds, fermentation products, cell extracts; plant extracts, animal tissue extracts, plasma, etc. These compounds may be either novel compounds or publicly known compounds.

The test compounds which are preferably used are compounds designed to bind to the ligand-binding pocket, based on the atomic coordinate and the position of the ligand-binding pocket in the active site of the receptor protein of the present invention. The atomic coordinate and the position of the ligand-binding pocket in the active site of the receptor protein of the present invention can be determined by publicly known methods or modifications thereof.

The kit for screening the compound or its salt that alters the binding properties of the ligand to the receptor protein, etc. of the present invention is a kit comprising the receptor protein, etc. of the present invention, a cell containing the receptor protein, etc. of the present invention, or a membrane fraction of the cell containing the receptor protein, etc. of the present invention, and the like.

Examples of the screening kit of the present invention include the following.

1. Reagent for screening

(1) Assay buffer and wash buffer
Hanks' balanced salt solution (manufactured by Gibco, Inc.) supplemented with 0.05% bovine serum albumin (manufactured by Sigma, Inc.)
The solution is sterilized by filtration through a 0.45 μm filter, and stored at 4°C or may be prepared at use.
(2) G protein-coupled receptor preparation
CHO cells wherein the receptor protein of the present invention has been expressed are passaged in a 12-well plate at a density of $5 \times 10^5$ cells/well followed by culturing at 37°C under 5% $CO_2$ and 95% air for 2 days.
(3) Labeled ligand
Ligand labeled with [$^3$H], [$^{125}$I], [$^{14}$C], [$^{35}$S], etc. commercially available
An aqueous solution of the substance is stored at 4°C or -20°C, and diluted to 1 μM with the assay buffer upon use.
(4) Ligand standard solution

The ligand is dissolved in and adjusted to 1 mM with PBS containing 0.1% bovine serum albumin (manufactured by Sigma, Inc.) and stored at -20°C.
2. Assay method

(1) CHO cells wherein the receptor protein of the present invention has been expressed are cultured in a 12-well culture plate and washed twice with 1 ml of the assay buffer, and 490 μl of the assay buffer is added to each well.
(2) After adding 5 μl of $10^{-3}$ - $10^{-10}$ M test compound solution, 5 μl of the labeled ligand is added to the mixture, and the cells are incubated at room temperature for an hour. To determine the amount of the non-specific binding, 5 μl of $10^{-3}$ M ligand is added in place of the test compound.

(3) The reaction solution is removed, and the wells are washed 3 times with the washing buffer. The labeled ligand bound to the cells is dissolved in 0.2N NaOH-1% SDS, and mixed with 4 ml of liquid scintillator A (manufactured by Wako Pure Chemical Industries, Ltd.).

(4) The radioactivity is measured using a liquid scintillation counter (manufactured by Beckman Co.), and the percent maximum binding (PMB) is calculated by the equation below.

$$PMB = [(B - NSB)/(B_0 - NSB)] \times 100$$

PMB : Percent maximum binding
B : Value obtained in the presence of a test compound
NSB : Non-specific binding
Bo : Maximum binding

A specific method to determine whether a compound or its salt that alters the binding properties of the ligand with the receptor protein, etc. of the present invention is an agonist or antagonist may be performed in accordance with either (i) or (ii) below.

(i) Binding assay as given for the screening methods (1) to (3) described above is performed to obtain a compound that alters the binding properties (especially inhibits the binding) of the ligand to the receptor protein, etc. of the present invention. Then, it is determined if the compound has the aforesaid cell stimulating activities described above mediated by the receptor protein, etc. of the present invention. The compound or its salt, which has the cell stimulating activities, is an agonist, whereas the compound or its salt having no such activities is an antagonist.

(ii) (a) A test compound is brought in contact with a cell containing the receptor protein of the present invention and the aforesaid cell stimulating activities mediated by the receptor protein of the present invention are assayed. The compound or its salt having the cell stimulating activities is an agonist.

(b) The cell stimulating activities mediated by the receptor protein of the present invention are assayed and compared between the case where the compound (e.g., ligand or agonist to the receptor protein of the present invention, etc.) that activates the receptor protein of the present invention is brought in contact with a cell containing the receptor protein of the present invention and the case where the compound that activates the receptor protein of the present invention and a test compound are brought in contact with a cell containing the receptor protein of the present invention. The compound or its salt that can decrease the cell stimulating activities by the compound, which activates the receptor protein of the present invention, is an antagonist.

The compound or its salt, which is obtained by using the screening methods or the screening kits of the present invention, is the compound that alters the binding properties of the ligand to the receptor protein, etc. of the present invention. Specifically, the compound includes: (1) a compound having the cell-stimulating activity mediated by the receptor protein of the present invention (so-called agonist to the receptor protein of the present invention); (2) a compound having no cell stimulating activity (so-called antagonist to the receptor protein of the present invention); (3) a compound that potentiates the binding affinity of the ligand to the receptor protein of the present invention; or (4) a compound that reduces the binding affinity of the ligand to the receptor protein of the present invention.

These compounds may be peptides, proteins, non-peptide compounds, synthetic compounds, fermentation products, etc. These compounds may be novel compounds or publicly known compounds.

Since the agonists to the receptor protein, etc. of the present invention have the same physiological activities as the aforesaid ligand to the receptor protein, etc. of the present invention has, the agonists are useful as safe and low toxic medicaments, correspondingly to the physiological activities.

The antagonists to the receptor protein, etc. of the present invention can suppress the physiological activities possessed by the aforesaid ligand to the receptor protein, etc. of the present invention, and are thus useful as safe and low toxic medicaments to suppress the physiological activities.

The compound that potentiates the binding affinity of the ligand to the receptor protein of the present invention is useful as a safe and low toxic medicament to potentiate the physiological activities possessed by the ligand to the receptor protein, etc. of the present invention.

The compound that reduces the binding affinity of the ligand to the receptor protein of the present invention is useful as a safe and low toxic medicament to reduce the physiological activities of the ligand on the receptor protein of the present invention thereby to suppress the physiological activities of the ligand possessed by the ligand to the receptor protein, etc. of the present invention.

Specifically, the compound or its salt, which is obtained by using the screening methods or screening kits of the present invention, can be used as an agent for the prevention and/or treatment of, e.g., obesity, mellitus diabetes, immune system disorders, liver diseases, alimentary disorders or tissue damages in cosmetic surgery.

(7) Preventive and/or Therapeutic Agent for Various Disorders Comprising Compound (Agonist, Antagonist, Inhibitor, Activator, etc.) That Alters Binding Properties of Receptor Protein of Invention to Ligand

The compounds (agonists, antagonists, inhibitors or activators) that alter the binding properties of the receptor protein of the present invention and the ligand or the aforesaid ligand for the receptor protein of the present invention can be used as agents for the prevention and/or treatment of disorders associated with dysfunction of the receptor protein of the present invention, for example, obesity, mellitus diabetes, immune system disorders, liver disorders, alimentary disorders or tissue damages in cosmetic surgery.

Where these compounds or ligands are used as agents for the prevention and/or treatment of disorders associated with dysfunction of the receptor protein of the present invention, they can be prepared into pharmaceutical preparations in a conventional manner as described above.

(8) Quantification of Protein, etc. Using Antibody of the Present invention, Diagnostic Agent Comprising Antibody of Invention, and Method for Diagnosis Using the Same

The antibody of the present invention is capable of specifically recognizing the receptor protein, etc. of the present invention, and thus can be used for quantification of the receptor protein, etc. of the present invention in a test fluid, in particular, for quantification including sandwich immunoassay, competitive method, immunometric method, nephrometry, and the like.

Special conditions or operations do not need to set forth in applying any of these immunoassay methods to the assay methods of the present invention. It may be sufficient to construct a measurement system for the receptor protein of the present invention or its salt by supplementing ordinary technical considerations of a skilled person to conventional conditions and operations for the individual methods. With respect to details of these conventional technical means, a variety of reviews, reference books, etc. may be referred to [see, for example, Irie, Hiroshi ed. Radioimmunoassay (Kodansha, 1974), Irie, Hiroshi ed. Radioimmunoassay Continued (Kodansha, 1979), Ishikawa, Eiji et al ed. Enzyme Immunoassay (Igaku Shoin, 1978), Ishikawa, Eiji et al ed. Enzyme Immunoassay 2.sup.nd edition (Igaku Shoin, 1982), Ishikawa, Eiji et al ed. Enzyme Immunoassay 3.sup.rd edition (Igaku Shoin, 1987), Methods in Enzymology Vol. 70 Immunochemical Techniques (Part A), Vol. 73 Immunochemical Techniques (Part B), Vol. 74 Immunochemical Techniques (Part C), Vol. 84 Immunochemical Techniques (Part D: Selected Immunoassays), Vol. 92 Immunochemical Techniques (Part E: Monoclonal Antibodies and General Immunoassay Methods), and Vol. 121 Immunochemical Techniques (Part I: Hybridoma Technology and Monoclonal Antibodies) (all Academic Press), WO publication 00/14227 page 39, line 25 through page 42, line 8, EP 1111047A2, paragraph [0115], page 19, line 35 through page 20, line 47].

As described above, the receptor protein of the present invention or its salt can be quantified with high sensitivity by using the antibodies of the present invention.

Moreover, various diseases associated with dysfunction of the receptor protein of the present invention can be diagnosed by using the antibodies of the present invention, whereby various diseases associated with dysfunction of the receptor protein of the present invention can be diagnosed.

For example, if an increased or decreased level of the receptor protein is detected by quantifying the level of the receptor protein of the present invention using the antibodies of the present invention, it can be diagnosed that it is highly likely to suffer from, for example, disease caused by the dysfunction or overexpression of the said receptor protein, such as obesity, diabetes mellitus, immune system disorders, liver disorders, alimentary disorders, etc.; or it is highly likely to suffer from these diseases in the future.

The antibodies of the present invention can also be used to specifically detect the receptor protein, etc. of the present invention, which is present in a test specimen such as body fluids, tissues, etc. They can also be used for preparing an antibody column to be used for purifying the receptor protein, etc. of the present invention, for detecting the receptor protein, etc. of the present invention in various fractions during purification, for analyzing the behavior of the receptor protein, etc. of the present invention in cells under test, and so on.

(9) Method of Screening Compound that Alters Level of Receptor Protein of Invention or its Partial Peptide in Cell Membranes

The antibody of the present invention is capable of specifically recognizing the receptor protein of the present invention, its partial peptide or salts thereof and thus can be used for screening the compound that alters the amount of the receptor protein of the present invention or its partial peptide in the cell membrane.

That is, the present invention provides, for example, the following methods:

(i) a method of screening the compound that alters the amount of the receptor protein of the present invention or its partial peptide in a cell membrane, which comprises quantifying the receptor protein of the present invention or its partial peptide contained in a cell membrane fraction isolated after disrupting a) blood, b) a particular organ or c) tissues or cells isolated from the organ of non-human mammal;

(ii) a method of screening the compound that alters the amount of the receptor protein of the present invention or its partial peptide in a cell membrane, which comprises disrupting transformants, etc. expressing the receptor protein of the present invention or its partial peptide, isolating the cell membrane fraction and quantifying the receptor protein of the present invention or its partial peptide contained in the cell membrane fraction;

(iii) a method of screening the compound that alters the amount of the receptor protein of the present invention or its partial peptide in a cell membrane, which comprises preparing a) blood, b) a particular organ or c) a slice of tissues or cells, etc. isolated from the organ of non-human mammal and quantifying the staining intensity of said receptor protein on the cell surface using immunostaining assay thereby to confirm said protein in the cell membrane; and,

(iv) a method of screening the compound that alters the amount of the receptor protein of the present invention in a cell membrane, which comprises preparing a slice of a transformant expressing the receptor protein of the present invention and quantifying the staining intensity of said receptor protein or its partial peptide on the cell surface using the immunostaining assay thereby to confirm said protein or its partial peptide on the cell membrane.

Specifically, the receptor protein of the present invention or its partial peptide contained in the cell membrane fraction can be quantified as follows.

(i) Normal or disease models of non-human mammals (e.g., mice, rats, rabbits, sheep, swine, bovine, cats, dogs, monkeys, etc., more specifically, tumor-bearing mice, etc.) receive a drug (e.g., an anticancer agent, etc.) or physical stress (e.g., soaking stress, electric shock, light and darkness, low temperature, etc.), and the blood, particular organs (e.g., brain, lung, colon, prostate, etc.), or tissues or cells isolated from the organs are obtained after a specified period of time. The obtained organs, tissues, cells or the like are suspended in, for example, an appropriate buffer (e.g., Tris hydrochloride buffer, phosphate buffer, HEPES buffer, etc.), etc., and the organs, tissues or cells are disrupted, and the cell membrane fraction is obtained using surfactants (e.g., Triton-X 100™, Tween 20™) and further using techniques such as centrifugal separation, filtration, column fractionation, etc.

The cell membrane fraction means a fraction abundant in cell membrane obtained by cell disruption and subsequent fractionation by publicly known methods. Cell disruption methods include cell squashing using a Potter-Elvehjem homogenizer, disruption using a Waring blender or Polytron (manufactured by Kinematica Inc.), disruption by ultrasonication, disruption by cell spraying through thin nozzles under an increased pressure using a French press, or the like. Cell membrane fractionation is effected mainly by fractionation using a centrifugal force, such as centrifugation for fractionation and density gradient centrifugation. For example, cell disruption fluid is centrifuged at a low speed (500 rpm to 3,000 rpm) for a short period of time (normally about 1 to about 10 minutes), the resulting supernatant is then centrifuged at a higher speed (15,000 rpm to 30,000 rpm) normally for 30 minutes to 2 hours. The precipitate thus obtained is used as the membrane fraction. The membrane fraction is rich in the receptor protein, etc. expressed and membrane components such as cell-derived phospholipids, membrane proteins, etc.

The receptor protein of the present invention or its partial peptide contained in the cell membrane fraction can be quantified by, for example, the sandwich immunoassay, western blot analysis, etc. using the antibody of the present invention.

The sandwich immunoassay can be performed as described above, and the western blot can be performed by publicly known methods.

(ii) Transformants expressing the receptor protein of the present invention or its partial peptide are prepared by the method described above, and the receptor protein of the present invention or its partial peptide contained in the cell membrane fraction can be quantified.

The compound that alters the amount of the receptor protein of the present invention or its partial peptide in cell membranes can be screened as follows.

(i) A test compound is administered to normal or disease models of non-human mammals at a specified period of time before (30 minutes to 24 hours before, preferably 30 minutes to 12 hours before, more preferably 1 hour to 6 hours before), at a specified time after (30 minutes to 3 days after, preferably 1 hour to 2 days after, more preferably 1 hour to 24 hours after) they receive a drug or physical stress, or simultaneously with a drug or physical stress. At a specified time (30 minute to 3 days, preferably 1 hour to 2 days, more preferably 1 hour to 24 hours) after administration of the test compound, the amount of the receptor protein of the present invention or its partial peptide in the cell membranes can be quantified for screening.

(ii) Transformants are cultured in a conventional manner and a test compound is mixed in the culture medium. After a specified time (after 1 day to 7 days, preferably after 1 day to 3 days, more preferably after 2 to 3 days), the amount of the receptor protein of the present invention or its partial peptide in the cell membranes can be quantified.

Specifically, the receptor protein of the present invention or its partial peptide contained in cell membrane fractions is confirmed as follows.

(iii) Normal or disease models of non-human mammals (e.g., mice, rats, rabbits, sheep, swine, bovine, cats, dogs, monkeys, etc., more specifically, Alzheimer disease model rats, mice, rabbits, etc.) are administered with a drug (e.g., an antidementia drug, hypotensive agent, anti-tumor agent, antiobesity agent, etc.) or physical stress (e.g., soaking stress, electric shock, light and darkness, low temperature, etc.) or the like, and blood or particular organ (e.g., brain, lung, colon, etc.), or the tissues or cells isolated from the organ are obtained after a specified period of time. Tissue slices are prepared from the thus obtained organs, tissues, cells, etc. in a conventional manner followed by immunostaining using the antibody of the present invention. The staining intensity of said receptor protein on the cell surface is quantified to confirm said protein on the cell membrane, whereby the amount of the receptor protein of the present invention or its partial peptide on the cell membrane can be confirmed quantitatively or qualitatively.

(iv) The confirmation can also be made in a similar manner, using transformants, etc., which express the receptor protein of the present invention or its partial peptide.

The compounds or their salts obtained by the screening methods of the present invention are compounds that have the action of altering the amount of the receptor protein of the present invention or its partial peptide in cell membranes. Specifically, these compounds are: (a) compounds that increase the amount of the receptor protein of the present invention or its partial peptide in cell membranes thereby to potentiate the receptor-mediated cell stimulating activities and (b) compounds that decrease the amount of the receptor protein of the present invention or its partial peptide in the cell membranes thereby to attenuate the cell stimulating activities.

The compounds may be peptides, proteins, non-peptide compounds, synthetic compounds, fermentation products, etc. These compounds may be novel compounds or publicly known compounds.

The compound that potentiates the cell stimulating activities is useful as a safe and low toxic medicament for potentiating the physiological activities of the receptor protein, etc. of the present invention.

The compound that attenuates the cell stimulating activities is useful as a safe and low toxic medicament to reduce the physiological activity of the receptor protein, etc. of the present invention.

(10) Marker for Somatic Stem Cell

The cholecystokinin receptor having the amino acid sequence represented by SEQ ID NO: 1 is specifically expressed in somatic stem cells, especially in bone marrow-derived mesenchymal stem cells. Therefore, the compound capable of binding to the protein comprising the same or substantially the same amino acid sequence as the amino acid sequence represented by SEQ ID NO: 1 can be a marker for somatic stem cells. Since such a marker can bind to the cholecystokinin receptor to label somatic stem cells having its receptor, especially bone marrow-derived mesenchymal stem cells, the marker can be used for identification, selection or concentration of such cells. As used herein, the compound capable of binding to the protein comprising the same or substantially the same amino acid sequence as the amino acid sequence represented by SEQ ID NO: 1 includes, e.g., an antibody capable of recognizing such a protein, a ligand, etc. Cholecystokinin is a ligand of the cholecystokinin receptor, and can be employed as a marker substance used herein. To become an identifiable marker, the substance is tagged with a label substance. Such a label substance is appropriately chosen from fluorescent substances such as fluorescein isothiocyanate, phycobiliprotein, rare earth metal chelates, dansyl chloride, tetramethyl rhodamine isothiocyanate, etc.; radioisotopes such as $^{3}H$, $^{14}C$, $^{125}I$, $^{131}I$, etc.

(11) Method of Differentiation-Inducing Mesenchymal Stem Cell

The present invention further provides a method of differentiation-inducing human mesenchymal stem cells into osteoblasts is also characterized in culturing human mesenchymal stem cells on a serum-free medium. Specifically, the method is characterized by using a serum-free medium containing oncostatin M.

The differentiation induction of mesenchymal stem cells can be effected as follows.

A maintenance medium may be any medium, as long as it can maintain cells but desirably it includes, for example, Dulbecco's Modified Eagle Medium (DMEM, Invitrogen) containing 1.0 g/l of glucose and supplemented with 10% fetal calf serum (Stem Cell Technologies), 2 mM L-glutamine (Invitrogen), 100 U/ml penicillin and 100 μg/ml

streptomycin. As a differentiation medium, there can be used the maintenance medium supplemented with 0.1 μM dexamethasone, 0.05 mM ascorbic acid and 10 mM β-glycerophosphate (=10% FBS-containing differentiation medium) or calf fetal serum-free maintenance medium supplemented with the three components described above (= serum-free differentiation medium). Serum-free medium supplemented with oncostatin M for cell differentiation induction is particularly preferred. Specifically, incubation and differentiation/induction can be performed as follows. Human mesenchymal stem cells are plated on a culture plate, e.g., a 24-well plate at 1 x $10^3$ to 1 x $10^4$/well, preferably at 6 x $10^3$/well, and incubated at 37°C overnight. After incubation, the medium is exchanged with a differentiation medium supplemented with 1 ng/ml to 10 ng/ml, preferably 10 ng/ml of oncostatin M and the exchange is made, e.g., every four days. The differentiation induction can be confirmed, e.g., by quantifying the accumulated calcium level per well on Day 10 after the incubation (Calcium C Test, Wako Pure Chemicals).

(12) Differentiation Inducer

The present invention provides an oncostatin M-containing cell differentiation inducer for cell-free medium, especially a cell differentiation inducer for cell-free medium that differentiates and induces human mesenchymal stem cells into osteoclasts. Oncostatin M can be added to the medium as it is or after dissolving in an appropriate buffer which does not interfere incubation, e.g., in 0.1%BSA-containing PBS or in an appropriate solvent such as methanol. The addition amount which can be used is in an amount of 1 ng/ml to 20 ng/ml, preferably 1 ng/ml to 15 ng/ml, more preferably 1 ng/ml to 10 ng/ml, and most preferably 10 ng/ml.

(Indication of Abbreviations)

As used herein, the codes of bases, amino acids, etc. are denoted in accordance with the IUPAC-IUB Commission on Biochemical Nomenclature or by the common codes in the art, examples of which are shown below. For amino acids that may have the optical isomer, L form is presented unless otherwise indicated.

| | |
|---|---|
| DNA : | deoxyribonucleic acid |
| cDNA : | complementary deoxyribonucleic acid |
| a or A : | adenine |
| t or T : | thymine |
| g or G : | guanine |
| c or C : | cytosine |
| u or U | :uracyl |
| RNA : | ribonucleic acid |
| mRNA : | messenger ribonucleic acid |
| dATP : | deoxyadenosine triphosphate |
| dTTP : | deoxythymidine triphosphate |
| dGTP : | deoxyguanosine triphosphate |
| dCTP : | deoxycytidine triphosphate |
| ATP | : adenosine triphosphate |
| Gly | : glycine |
| Ala | : alanine |
| Val | : valine |
| Leu | : leucine |
| Ile | : isoleucine |
| Ser | : serine |
| Thr | : threonine |
| Cys | : cysteine |
| Met | : methionine |
| Glu | : glutamic acid |
| Asp | : aspartic acid |
| Lys | :lysine |
| Arg | : arginine |
| His | : histidine |
| Phe | : phenylalanine |
| Tyr | : tyrosine |
| Trp | : tryptophan |
| Pro | : proline |

Asn : asparagine
Gln : glutamine
pGlu : pyroglutamic acid

Substituents, protecting groups and reagents frequently used in this specification are presented as the codes below.

[SEQ ID NO: 1]
This shows the amino acid sequence of the human cholecystokinin receptor.
[SEQ ID NO: 2]
This shows the base sequence of DNA encoding the human cholecystokinin receptor

EXAMPLES

Hereinafter, the present invention will be described in more detail but the scope of the present invention is not deemed to be limited thereto.

EXAMPLE 1

(Detection of Receptor Expressed in Human Mesenchymal Stem Cell)

Total RNA fraction was prepared from commercially available human mesenchymal stem cells in accordance with a publicly known method, and cDNA was then synthesized according to a general method (see, e.g., Molecular Cloning Third Edition: A Laboratory Manual (Sambrook, et al.)). Using the Real Time PCR system from Applied Biosystems, the expression level of G protein-coupled receptor family mRNA was quantified, and cholecystokinin receptor type A was found to be expressed in human mesenchymal stem cells by approximately 71,000 copies as the copy number of mRNA contained in 25 ng of total RNA.

EXAMPLE 2

(Regulation of Differentiation into Adipocyte Using Ligand for Receptor Expressed in Human Mesenchymal Stem Cell)

Human mesenchymal stem cells were incubated in a 24-well culture plate until the cells became confluent. Then, the medium was exchanged with a differentiation induction medium prepared by adding cholecystokinin (100 nM) to high-glucose DMEM medium supplemented with insulin, dexamethasone, isobutylmethylxanthine, indomethacin, antibiotic and 10% fetal bovine serum, and incubation was continued for 3 days. For control, a cholecystokinin-free differentiation induction medium was used. After incubation was conducted for 3 days in a high-glucose DMEM medium (adipocyte maintenance medium) supplemented only with insulin, antibiotic and 10% fetal bovine serum, the same treatments (the induction and the maintenance, respectively, for 3 days) were repeated. Accumulated intracellular lipids were visualized by staining with Oil Red O dye.
The results obtained are shown in FIG. 1. As shown in FIG 1, the accumulated intracellular lipids are detected by staining with Oil Red O dye, which reveals that the lipid accumulation is enhanced by reacting cholecystokinin (CCK: 100 nM) simultaneously, when compared to control.

EXAMPLE 3

(Effect of Oncostatin M in Osteoblast Differentiation Induction from Human Mesenchymal Stem Cell)

Human mesenchymal stem cells were purchased from Cambrex, Inc. Dulbecco's Modified Eagle Medium (DMEM, Invitrogen) containing 1.0 g/l of glucose and supplemented with 10% fetal bovine serum (Stem Cell Technologies), 2 mM L-glutamine (Invitrogen), 100 U/ml penicillin and 100 μg/ml streptomycin was used as a maintenance medium. The maintenance medium supplemented with 0.1 μM dexamethasone, 0.05 mM ascorbic acid and 10 mM β-glycerophosphate (= 10% FBS-containing differentiation medium) or fetal bovine serum-free maintenance medium supplemented with the three components above was used as a differentiation medium (= serum-free differentiation medium). After human mesenchymal stem cells were plated overnight on a 24-well plate at a density of 6 x $10^3$/well, the medium was replaced with a differentiation medium supplemented with Oncostatin M at each density (10 ng/ml, 0 ng/ml). Subsequently, the medium was exchanged every 4 days, and the accumulated calcium level per well was quantified on day 10 of the incubation (Calcium C-Test, Wako Pure Chemical). As shown in FIG 2, the results indicate that calcium was more accumulated in the 10% FBS-containing differentiation medium than in the serum-free differentiation medium under the conditions where oncostatin M was not added, whereas under the conditions where oncostatin M (10 μg/ml) was added, marked accumulation of calcium was observed, irrespective of the presence or absence of FBS, when compared to the 10% FBS-containing differentiation medium where oncostatin M was not added. These results indicate that oncostatin M is useful as the differentiation induction factor in the differentiation induction system of osteoblasts from human mesenchymal stem cells in serum-free medium.

INDUSTRIAL APPLICABILITY

According to the methods of the present invention for screening the genes associated with cell regeneration, growth or differentiation, the genes associated with cell regeneration, growth or differentiation can be efficiently identified in a few steps in a cell having the regeneration, differentiation or growth capability. Further according to the methods of the present invention for screening the regulators for cell regeneration, growth or differentiation, the regulators for cell regeneration, growth or differentiation can be efficiently obtained as compared to conventional methods.

The regulators for cell regeneration, growth or differentiation identified by these methods can regulate cell regeneration, growth or differentiation, and are thus useful as agents for the prevention and/or treatment of disorders or injuries, for example, diseases associated with cell regeneration, growth or differentiation, for example, neurological disorders, inflammatory disorders, circulatory disorders, myocardial infarction, heart failure, cancer, obesity, diabetes mellitus, immune disorders, liver/gallbladder disorders, alimentary disorders, heat burn, fracture, osteoarthritis, periodontal disease, alopecia, etc.

Further according to the diagnostic agents, kits for diagnosis and methods for diagnosis of the present invention, which are used for diagnosis of diseases associated with cell regeneration, growth or differentiation, it is advantageous in that the diseases associated with cell regeneration, growth or differentiation can be diagnosed in a simple manner.

## Claims

**1.** A method of screening a gene associated with the regeneration, growth or differentiation of a cell, which comprises the step of quantitatively analyzing expression levels of multiple genes collectively in a cell having a regeneration, differentiation or growth capability to thereby identify the gene associated with the regeneration, growth or differentiation of the cell.

**2.** The method according to claim 1, which comprises the step of quantitatively analyzing expression levels of multiple genes collectively in a cell having a regeneration, differentiation or growth capability to thereby identify one or more genes that show higher expression levels in the cell or are specific in expression as compared to the other cells; and, the step of contacting at least one candidate substance capable of regulating the function of the identified genes or their gene products with the cell and comparing changes before and after the contact.

**3.** The screening method according to claim 1, wherein the cell having a regeneration, differentiation or growth capability is selected from an embryonic stem cell, a somatic stem cell, a primary culture cell and a cell line from human or warm-blooded animal.

**4.** The method according to claim 1, wherein the multiple genes are a group of genes selected from a G protein-coupled receptor gene family; a nuclear receptor gene family; a tyrosine kinase-type receptor gene family; a transcription factor; a gene family related to any one of a protein kinase, a protein phosphatase, a protease, an ATPase, a GTPase, a DNA-bound protein, a cell adhesion factor and its receptor, an ion channel, a transporter, an extracellular matrix component, an intracellular cytoskeleton component, a cell growth factor, a cytokine, a neurotrophic factor, a physiologically active peptide, a hormone, an oxidase or reductase, a hydrolase, a hydroxylase, a methylase or demethylase, a transferase, a ribosome-constituting protein and a histocompatible antigen protein group; and a group of genes which act directly or indirectly on the above genes to regulate their activities.

**5.** The method according to claim 1, wherein the multiple genes are the group of genes selected from the G protein-coupled receptor gene family, the nuclear receptor gene family and the protein kinase-type receptor gene family.

**6.** The method according to claim 1, wherein an mRNA sample suspected of containing multiple target mRNAs obtained from a cell having a regeneration, differentiation or growth capability is brought in contact with each amplification reagent comprising a pair of primers corresponding to each target mRNA, respectively, at each reaction site of a reactor having multiple reaction sites for amplification; and the amounts of amplification products formed are measured to thereby identify the genes associated with the regeneration, growth or differentiation of the cell.

**7.** The method according to claim 6, wherein the reactor is a plate having multiple wells as the reaction sites.

**8.** The method according to claim 7, wherein the plate is a 96-well or 384-well plate.

**9.** The method according to claim 6, wherein 10 to 800 pairs of primers are used.

**10.** The method according to claim 6, wherein 10 to 300 pairs of primers are used.

**11.** The method according to claim 6, wherein the amplification is performed by a polymerase chain reaction.

**12.** The method according to claim 6, wherein the measurement of the amount of the amplification product formed is performed using a probe complementary or substantially complementary to the amplification product

**13.** The method according to claim 12, wherein the probe is a probe hybridizable to mRNA and cDNA.

**14.** The method according to claim 12, wherein the probe is a fluorescence-labeled probe.

**15.** The method according to claim 13, wherein an mRNA showing increased or decreased expression in the mRNA sample is identified and a gene encoding the mRNA is identified as the gene associated with the regeneration, growth or differentiation of the cell.

**16.** A method of screening a regulator for the regeneration, growth or differentiation of a cell, which comprises the step of quantitatively analyzing expression levels of multiple genes collectively in a cell having a regeneration, differentiation or growth capability to thereby identify one or more genes that show higher expression levels in the cell or are specific in expression as compared to the other cells; and, the step of contacting at least one candidate substance which can regulate the function of the identified genes or their gene products with the cell and comparing changes before and after the contact.

**17.** A method of regulating the regeneration, growth or differentiation of a cell comprising the gene product of the gene identified by the screening method according to claim 1, which comprises inhibiting or activating the gene product.

**18.** The method according to claim 17, wherein the cell is an embryonic stem cell, a somatic stem cell, a mesenchymal stem cell, a spleen cell, a neuronal cell, a glial cell, a pancreatic cell, a cartilage cell, a bone cell, a bone marrow cells, a mesangial cell, an epithelial cell, an epidermal cell, an endothelial cell, a fibroblast cell, a fiber cell, a muscle cell, a fat cell, an immune cell, an vascular endothelial cell, an endothelial progenitor cell or a hematopoietic cell.

**19.** A method of regenerating an organ, which comprises using the method according to claim 17.

**20.** The method according to claim 19, wherein the organ is brain, each part of the brain, cornea, spinal marrow, pituitary, stomach, pancreas, kidney, liver, genital gland, thyroid gland, gallbladder, bone marrow, adrenal, skin, muscle, lung, digestive tract, blood vessel, heart, thymus gland, spleen, submandibular gland, peripheral blood, peripheral blood cell, prostate gland, testicle, testis, ovary, placenta, uterus, tooth, bone, joint or skeletal muscle.

**21.** The method according to claim 17, which is a method of treating a disease associated with the regeneration, growth or differentiation of the cell.

**22.** The method according to claim 17, wherein the disease is a central system disorder/neurological disorder, an inflammatory disorder, a circulatory disorder, cancer, obesity, diabetes, an immune disorder, a liver/gallbladder disorder, an alimentary disorder, heat burn, fracture, osteoarthritis, periodontal disease or alopecia.

**23.** A medicament comprising a protein comprising the same or substantially the same amino acid sequence as the amino acid sequence represented by SEQ ID NO: 1, or a DNA encoding the protein.

**24.** A method of regulating the regeneration, growth or differentiation of a cell comprising a protein comprising the same or substantially the same amino acid sequence as the amino acid sequence represented by SEQ ID NO: 1, which comprises inhibiting or activating the protein.

**25.** The medicament according to claim 23, which is a regulator for the regeneration, growth or differentiation of a cell.

26. A regulator for the regeneration, growth or differentiation of a cell comprising a protein comprising the same or substantially the same amino acid sequence as the amino acid sequence represented by SEQ ID NO: 1, which comprises cholecystokinin or its analogue.

27. The regulator according to claim 26, which is capable of regulating the differentiation of a stem cell.

28. The regulator according to claim 27, which is capable of regulating the differentiation of a mesenchymal stem cell.

29. The regulator according to claim 26, which is capable of regulating the differentiation into a fat cell.

30. A cell, which is obtained by regulating the differentiation with the regulator according to claim 26.

31. A method of regulating the regeneration, growth or differentiation of a cell using the regulator according to claim 26.

32. A method of regenerating an organ, which comprises using the method according to claim 31.

33. A method of treating a disease associated with the regeneration, growth or differentiation of a cell, which comprises administering the regulator according to claim 26.

34. The method according to claim 33, wherein the disease is obesity, diabetes, an immune disorder, a liver disorder, an alimentary disorder, or tissue damages in plastic surgery.

35. A method of screening a compound capable of regulating the regeneration, growth or differentiation of a cell, which comprises using a protein comprising the amino acid sequence represented by SEQ ID NO: 1, or its partial peptide.

36. A kit for screening a compound capable of regulating the regeneration, growth or differentiation of a cell, which comprises using a protein comprising the amino acid sequence represented by SEQ ID NO: 1, or its partial peptide.

37. A marker for a somatic stem cell, which comprises a compound capable of binding to a protein comprising the same or substantially the same amino acid sequence as the amino acid sequence represented by SEQ ID NO: 1.

38. The marker according to claim 37, wherein a somatic stem cell is labeled with the compound, thereby the marker being usable for discriminating, selecting or condensing the cell.

39. The marker according to claim 37, which is capable of labeling the mesenchymal stem cell derived from bone marrow.

40. A method of treatment by compensating for the function lost due to a damage caused by transplantation or a disease, which comprises using the cell according to claim 30.

41. A method of screening a medicament candidate compound, which comprises using the cell according to claim 30.

42. A method of confirming the safety of a medicament candidate compound, which comprises using the cell according to claim 30.

43. A method of differentiation-inducing a human mesenchymal stem cell into an osteoblast cell, which comprises culturing the human mesenchymal stem cell on a serum-free medium.

44. The method according to claim 43, wherein oncostatin M is added to the serum-free medium.

45. A cell differentiation inducer for a serum free medium, which comprises oncostatin M.

46. The cell differentiation inducer according to claim 45, which differentiates and induces a human mesenchymal stem cell into an osteoblast cell.

Fig. 1

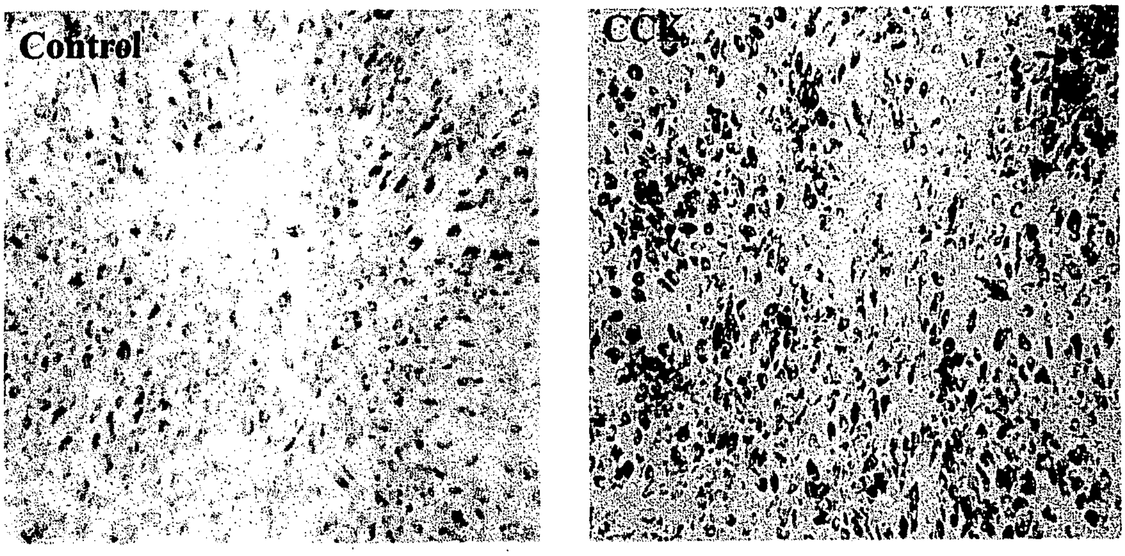

Fig. 2

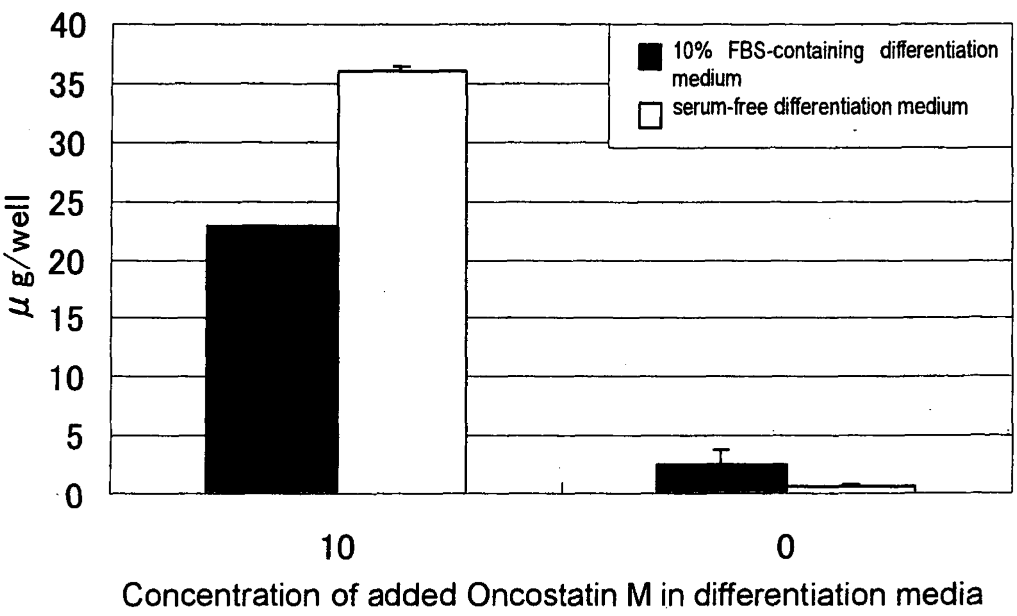
40
35
30
25
20
15
10
5
0
μg/well
10% FBS-containing differentiation medium
serum-free differentiation medium
10
0
Concentration of added Oncostatin M in differentiation media

## INTERNATIONAL SEARCH REPORT

International application No.
PCT/JP2005/013220

A. CLASSIFICATION OF SUBJECT MATTER
***C12Q1/68*** (2006.01), ***A61K31/517*** (2006.01), ***A61K35/48*** (2006.01), ***A61K45/00*** (2006.01), ***A16K48/00*** (2006.01), ***A61P37/00***(2006.01), ***A61P43/00***(2006.01), ***C07K14/705***(2006.01), ***C12N15/09***(2006.01),
According to International Patent Classification (IPC) or to both national classification and IPC

B. FIELDS SEARCHED
Minimum documentation searched (classification system followed by classification symbols)
***C12Q1/68*** (2006.01), ***A61K31/517*** (2006.01), ***A61K35/48*** (2006.01), ***A61K45/00*** (2006.01), ***A16K48/00*** (2006.01), ***A61P37/00***(2006.01), ***A61P43/00***(2006.01), ***C07K14/705***(2006.01), ***C12N15/09***(2006.01),

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)
CA/BIOSIS/MEDLINE/WPIDS(STN), JSTPlus(JOIS)

C. DOCUMENTS CONSIDERED TO BE RELEVANT

| Category* | Citation of document, with indication, where appropriate, of the relevant passages | Relevant to claim No. |
|---|---|---|
| Y | JP 2004-215503 A (Takeda Chemical Industries, Ltd.), 05 August, 2004 (05.08.04), Full text & WO 2003/052096 A1 & EP 1454982 A1 | 1-20 |
| Y | WO 2002/053592 A1 (Shionogi & Co., Ltd.), 11 July, 2002 (11.07.02), Full text & EP 1354890 A1 & US 2004/0110921 A1 | 1-20 |
| Y | JAISWAL Rama K. et al., Adult Human Mesenchymal Stem Cell Differentiatio to the Osteogenic or Adipogenic Lineage Is Regulated by Mitogen-activated Protein kinase, THE JOURNAL OF BIOLOGICAL CHEMISTRY, 275(13), 9645-9652(2000) | 1-20 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 05 October, 2005 (05.10.05) | 25 October, 2005 (25.10.05) |

| Name and mailing address of the ISA/ | Authorized officer |
|---|---|
| Japanese Patent Office | |
| Facsimile No. | Telephone No. |

Form PCT/ISA/210 (second sheet) (January 2004)

**INTERNATIONAL SEARCH REPORT**

International application No.
PCT/JP2005/013220

C (Continuation). DOCUMENTS CONSIDERED TO BE RELEVANT

| Category* | Citation of document, with indication, where appropriate, of the relevant passages | Relevant to claim No. |
|---|---|---|
| Y | WO 2002/045506 A1 (UNIVERSITY OF FLORIDA), 13 June, 2002 (13.06.02), Full text & US 2002/0115059 A1 | 1-20 |
| X<br>Y | CATLEDGE Shane A. et al., Mesenchymal stem cell adhesion and spreading on nanostructured biomaterials, JOURNAL OF NANOSCIENCE AND NANOTECHNOLOGY, 4(8), 986-989(2004) | 43<br>44,46 |
| X<br>Y | WO 2001/051616 A2 (GERON CORP.), 19 July, 2001 (19.07.01), & JP 2003-530828 A & JP 2003-111588 A & US 2002/0019046 A1 & EP 1250420 A2 | 45<br>44,46 |
| A | ULRICH Charles D. et al., MOLECULAR CLONING AND FUNCTIONAL EXPRESSION OF THE HUMAN GALLBLADDER CHOLECYSTOKININ A RECEPTOR, BIOCHEMICAL AND BIOPHYSICAL RESEARCH COMMUNICATIONS, 193(1), 204-211(1993) | 23-32,35-39, 41-42 |
| A | POVOSKI Stephen P. et al., Stimulatin of In Vivo Pancreatic Growth in the Rat Is Mediated Specifically by Way of Cholecystokinin-A Receptors, GASTRONTEROLOGY, 107, 1135-1146(1994) | 23-32,35-39, 41-42 |

Form PCT/ISA/210 (continuation of second sheet) (January 2004)

**INTERNATIONAL SEARCH REPORT**

International application No.
PCT/JP2005/013220

**Box No. II Observations where certain claims were found unsearchable (Continuation of item 2 of first sheet)**

This international search report has not been established in respect of certain claims under Article 17(2)(a) for the following reasons:

1. [X] Claims Nos.: 21, 22, 33, 34, 40
because they relate to subject matter not required to be searched by this Authority, namely:
Claims 21, 22, 33, 34 and 40 pertain to methods for treatment of the human body by therapy and thus relate to a subject matter which this International Searching Authority is not required, under the provisions of the PCT Rule 39.1(iv), to search.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. The inventions according to claims 1 to 15 relate to "a method of screening a gene participating in the regeneration, proliferation or differentiation of cells capable of regenerating, differentiating and proliferating".
2. The invention according to claim 16 relates to "a method of screening a cell regeneration-, proliferation- and differentiation-controlling agent".
3. The inventions according to claims 17 to 18 relate to "a method of controlling the regeneration, proliferation or differentiation of cells".
4. The inventions according to claims 19 to 20 relate to "a method of regenerating an organ". (Continued to extra sheet.)

1. [ ] As all required additional search fees were timely paid by the applicant, this international search report covers all searchable claims.
2. [X] As all searchable claims could be searched without effort justifying an additional fee, this Authority did not invite payment of any additional fee.
3. [ ] As only some of the required additional search fees were timely paid by the applicant, this international search report covers only those claims for which fees were paid, specifically claims Nos.:
4. [ ] No required additional search fees were timely paid by the applicant. Consequently, this international search report is restricted to the invention first mentioned in the claims; it is covered by claims Nos.:

**Remark on Protest**
[ ] The additional search fees were accompanied by the applicant's protest.
[ ] No protest accompanied the payment of additional search fees.

Form PCT/ISA/210 (continuation of first sheet (2)) (January 2004)

INTERNATIONAL SEARCH REPORT

International application No. PCT/JP2005/013220

Continuation of A. CLASSIFICATION OF SUBJECT MATTER
(International Patent Classification (IPC))
***C12N5/06*** (2006.01), ***C12Q1/02*** (2006.01), ***G01N33/15*** (2006.01), ***G01N33/50*** (2006.01)

(According to International Patent Classification (IPC) or to both national classification and IPC)

Continuation of B. FIELDS SEARCHED
Minimum documentation searched (International Patent Classification (IPC))
***C12N5/06*** (2006.01), ***C12Q1/02*** (2006.01), ***G01N33/15*** (2006.01), ***G01N33/50*** (2006.01)

Minimum documentation searched (classification system followed by classification symbols)

Continuation of Box No.III of continuation of first sheet(2)

1. The inventions according to claims 23 to 32, 35 to 39 and 41 to 42 relate to "use of the amino acid sequence represented by SEQ ID NO:1, etc. as a cell regeneration-, proliferation- and differentiation-controlling agent".
2. The inventions according to claims 43 to 46 relate to "cell differentiation on a serum-free medium".

Since there is no technical relationship involving one or more of the same or corresponding technical features among these invention groups, it does not appear that these invention groups are so linked as to form a single general inventive concept.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4683195 A
- US 4683202 A
- US 4965188 A
- US 5310652 A
- US 5322770 A
- US 5561058 A
- US 5641864 A
- US 5693517 A
- US 5455166 A
- US 5437990 A
- US 5409818 A
- US 5399491 A
- WO 9208800 A
- US 5210015 A
- US 5487972 A
- US 5804375 A
- JP 2001204483 A
- US 5219717 A
- US 5476774 A
- EP 0866071 A
- WO 0014227 A
- EP 1111047 A2


### Non-patent literature cited in the description


- *Genome Res,* October 1996, vol. 6 (10), 986-94
- *Genome Res.,* 06 October 1996, 986-94
- *Proceedings of the National Academy of Sciences U.S.A.,* 1991, vol. 88, 7276-7280
- *Molecular Medicine,* 2003, vol. 40, 144-151
- *Molecular Medicine,* 2003, vol. 40, 265-270
- *Nat. Med.,* 1999, vol. 5, 975-977
- **M. V. WILES.** Embryonic Stem Cell Differentiation in vitro. *Meth. Enzymol.,* 1993, vol. 225, 900
- *Meth. Cell Biol.,* 1997, vol. 75, 173
- *Large Scale Mammalian Cell Culture Curr. Opin. Biotechnol.,* 1997, vol. 8, 148
- *Molecular Medicine,* 2003, vol. 40, 2-8
- *J. Thorac. Cardiovasc. Surg.,* 1997, vol. 113, 10-18
- *Nat. Med.,* 2001, vol. 7, 430-436
- *Nature,* 2001, vol. 410, 701-705
- *J. Neurosci. Res.,* 2000, vol. 561, 364-370
- *Stroke,* 2001, vol. 32, 1005-1011
- *Science,* 2000, vol. 288, 1660-1663
- **M. BODANSZKY ; M.A. ONDETTI.** Peptide Synthesis. Interscience Publishers, 1966
- **SCHROEDER ; LUEBKE.** The Peptide. Academic Press, 1965
- **NOBUO IZUMIYA et al.** Peptide Gosei-no-Kiso to Jikken. Maruzen Co, 1975
- Seikagaku Jikken Koza. **HARUAKI YAJIMA ; SHUNPEI SAKAKIBARA.** Tanpakushitsu no Kagaku. 1977, vol. IV, 205
- Zoku Iyakuhin no Kaihatsu. Peptide Synthesis. Hirokawa Shoten, vol. 14
- New PCR and its application. *Jikken Igaku,* 1997, vol. 15 (7
- **J. SAMBROOK et al.** Molecular Cloning. Cold Spring Harbor Lab. Press
- **J. KAWAKAMI et al.** *Pharm. Tech. Japan,* 1992, vol. 8, 247
- *PHARM. TECH. JAPAN,* 1992, vol. 8, 395
- Antisense Research and Applications. CRC Press, 1993
- **KOEHLER ; MILSTEIN.** *Nature,* 1975, vol. 256, 495
- *Genomics,* 1989, vol. 5, 874-879
- *Proceedings of the National Academy of Sciences of the United States of America,* 1989, vol. 86, 2766-2770
- Radioimmunoassay. Kodansha, 1974
- Radioimmunoassay. Kodansha, 1979
- Enzyme Immunoassay. Igaku Shoin, 1978
- Enzyme Immunoassay. Igaku Shoin, 1982
- Enzyme Immunoassay. Igaku Shoin, 1987
- Immunochemical Techniques (Part A. Methods in Enzymology. vol. 70
- Immunochemical Techniques (Part B. METHODS IN ENZYMOLOGY. vol. 73
- Immunochemical Techniques (Part C. METHODS IN ENZYMOLOGY. vol. 74
- Immunochemical Techniques (Part D: Selected Immunoassays. METHODS IN ENZYMOLOGY. vol. 84
- Immunochemical Techniques (Part E: Monoclonal Antibodies and General Immunoassay Methods. METHODS IN ENZYMOLOGY. vol. 92

- Immunochemical Techniques (Part I: Hybridoma Technology and Monoclonal Antibodies. METHODS IN ENZYMOLOGY. Academic Press, vol. 121

SEQUENCE LISTING

<110> Takeda Pharmaceutical Company Limited

<120> A method of regulating cell functions

<130> G06-0113

<150> JP 2004-205877
<151> 2004-07-13

<150> JP 2004-319580
<151> 2004-11-02

<160> 2

<170> PatentIn version 3.1

<210> 1
<211> 428
<212> PRT
<213> Homo sapience

<400> 1

```
Met Asp Val Val Asp Ser Leu Leu Val Asn Gly Ser Asn Ile Thr Pro
1               5                   10                  15
Pro Cys Glu Leu Gly Leu Glu Asn Glu Thr Leu Phe Cys Leu Asp Gln
            20                  25                  30
Pro Arg Pro Ser Lys Glu Trp Gln Pro Ala Val Gln Ile Leu Leu Tyr
        35                  40                  45
Ser Leu Ile Phe Leu Leu Ser Val Leu Gly Asn Thr Leu Val Ile Thr
    50                  55                  60
Val Leu Ile Arg Asn Lys Arg Met Arg Thr Val Thr Asn Ile Phe Leu
65                  70                  75                  80
Leu Ser Leu Ala Val Ser Asp Leu Met Leu Cys Leu Phe Cys Met Pro
                85                  90                  95
Phe Asn Leu Ile Pro Asn Leu Leu Lys Asp Phe Ile Phe Gly Ser Ala
            100                 105                 110
Val Cys Lys Thr Thr Thr Tyr Phe Met Gly Thr Ser Val Ser Val Ser
        115                 120                 125
Thr Phe Asn Leu Val Ala Ile Ser Leu Glu Arg Tyr Gly Ala Ile Cys
    130                 135                 140
Lys Pro Leu Gln Ser Arg Val Trp Gln Thr Lys Ser His Ala Leu Lys
145                 150                 155                 160
Val Ile Ala Ala Thr Trp Cys Leu Ser Phe Thr Ile Met Thr Pro Tyr
                165                 170                 175
```

```
Pro Ile Tyr Ser Asn Leu Val Pro Phe Thr Lys Asn Asn Asn Gln Thr
            180                 185                 190
Ala Asn Met Cys Arg Phe Leu Leu Pro Asn Asp Val Met Gln Gln Ser
        195                 200                 205
Trp His Thr Phe Leu Leu Leu Ile Leu Phe Leu Ile Pro Gly Ile Val
    210                 215                 220
Met Met Val Ala Tyr Gly Leu Ile Ser Leu Glu Leu Tyr Gln Gly Ile
225                 230                 235                 240
Lys Phe Glu Ala Ser Gln Lys Lys Ser Ala Lys Glu Arg Lys Pro Ser
                245                 250                 255
Thr Thr Ser Ser Gly Lys Tyr Glu Asp Ser Asp Gly Cys Tyr Leu Gln
            260                 265                 270
Lys Thr Arg Pro Pro Arg Lys Leu Glu Leu Arg Gln Leu Ser Thr Gly
        275                 280                 285
Ser Ser Ser Arg Ala Asn Arg Ile Arg Ser Asn Ser Ser Ala Ala Asn
    290                 295                 300
Leu Met Ala Lys Lys Arg Val Ile Arg Met Leu Ile Val Ile Val Val
305                 310                 315                 320
Leu Phe Phe Leu Cys Trp Met Pro Ile Phe Ser Ala Asn Ala Trp Arg
                325                 330                 335
Ala Tyr Asp Thr Ala Ser Ala Glu Arg Arg Leu Ser Gly Thr Pro Ile
            340                 345                 350
Ser Phe Ile Leu Leu Leu Ser Tyr Thr Ser Ser Cys Val Asn Pro Ile
        355                 360                 365
Ile Tyr Cys Phe Met Asn Lys Arg Phe Arg Leu Gly Phe Met Ala Thr
    370                 375                 380
Phe Pro Cys Cys Pro Asn Pro Gly Pro Pro Gly Ala Arg Gly Glu Val
385                 390                 395                 400
Gly Glu Glu Glu Glu Gly Gly Thr Thr Gly Ala Ser Leu Ser Arg Phe
                405                 410                 415
Ser Tyr Ser His Met Ser Ala Ser Val Pro Pro Gln
            420                 425
```

<210> 2
<211> 1393
<212> DNA
<213> Homo sapience

<400> 2

```
cattagagga atgagccggg agtgagcaat tcaccagctc tccagcactt ggtggaaagc     60
agcaggcaag gatggatgtg gttgacagcc ttcttgtgaa tggaagcaac atcactcctc    120
cctgtgaact cgggctcgaa aatgagacgc ttttctgctt ggatcagccc cgtccttcca    180
aagagtggca gccagcggtg cagattctct tgtactcctt gatattcctg ctcagcgtgc    240
tgggaaacac gctggtcatc accgtgctga ttcggaacaa gcggatgcgg acggtcacca    300
acatcttcct cctctccctg gctgtcagcg acctcatgct ctgtctcttc tgcatgccgt    360
tcaacctcat ccccaatctg ctcaaggatt tcatcttcgg gagcgccgtt tgcaagacca    420
ccacctactt catgggcacc tctgtgagtg tatctacctt taatctggta gccatatctc    480
```

```
tagagagata tggtgcgatt tgcaaaccct tacagtcccg ggtctggcag acaaaatccc    540
atgctttgaa ggtgattgct gctacctggt gcctttcctt taccatcatg actccgtacc    600
ccatttatag caacttggtg ccttttacca aaaataacaa ccagaccgcg aatatgtgcc    660
gctttctact gccaaatgat gttatgcagc agtcctggca cacattcctg ttactcatcc    720
tctttcttat tcctggaatt gtgatgatgg tggcatatgg attaatctct ttggaactct    780
accagggaat aaaatttgag gctagccaga agaagtctgc taaagaaagg aaacctagca    840
ccaccagcag cggcaaatat gaggacagcg atgggtgtta cctgcaaaag accaggcccc    900
cgaggaagct ggagctccgg cagctgtcca ccggcagcag cagcagggcc aaccgcatcc    960
ggagtaacag ctccgcagcc aacctgatgg ccaagaaaag ggtgatccgc atgctcatcg   1020
tcatcgtggt cctcttcttc ttgtgctgga tgcccatctt cagcgccaac gcctggcggg   1080
cctacgacac cgcctccgca gagcgccgcc tctcaggaac ccccatttcc ttcatcctcc   1140
tcctgtccta cacctcctcc tgcgtcaacc ccatcatcta ctgcttcatg aacaaacgct   1200
tccgcctcgg cttcatggcc accttcccct gctgccccaa tcctggtccc ccaggggcga   1260
ggggagaggt gggggaggag gaggaaggcg ggaccacagg agcctctctg tccaggttct   1320
cgtacagcca tatgagtgcc tcggtgccac cccagtgaga tgtcccctga ccctccaccg   1380
cagaaggaag gca                                                      1393
```